# EUROPEAN PATENT APPLICATION

(11) **EP 1 889 840 A1**
(43) Date of publication of application: **20.02.2008**
(21) Application number: 06380204.5
(22) Date of filing: 19.07.2006
(51) Int. Cl.: C07D 261/20, C07D 307/83, C07D 491/22

(54) **Rearrangement of spirolactams**

(71) Applicant: LABORATORIOS DEL DR. ESTEVE, S.A., 08041 Barcelona (ES)
(72) Inventor: Noheda Marin, Pedro, I.Q.O.G, CSIC, 28006 Madrid (ES); Maroto Quintana, Sergio, I.Q.O.G, CSIC, 28006 Madrid (ES); Tabares Cantero, Nuria, I.Q.O.G, CSIC, 28006 Madrid (ES); Benito Arenas, Raul, I.Q.O.G, CSIC, 28006 Madrid (ES); Hojas Garcia, Elena, I.Q.O.G, CSIC, 28006 Madrid (ES)
(74) Representative: ABG Patentes, S.L.

(57) **Abstract**

The present invention refers to a process for rearranging a spirolactam (I) under acidic conditions to yield an oxabicyclo[4.3.0]none (II) or (III) or a 1-aza-2oxaspiro derivative (IV). The present invention also refers said oxabicyclo[4.3.0]none and 1-aza-2oxaspiro derivatives.

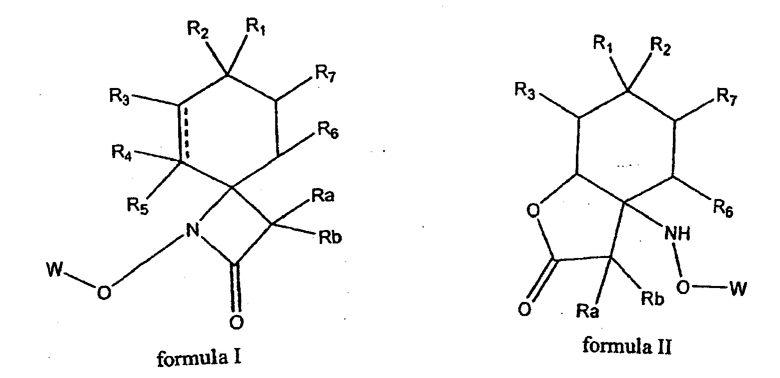

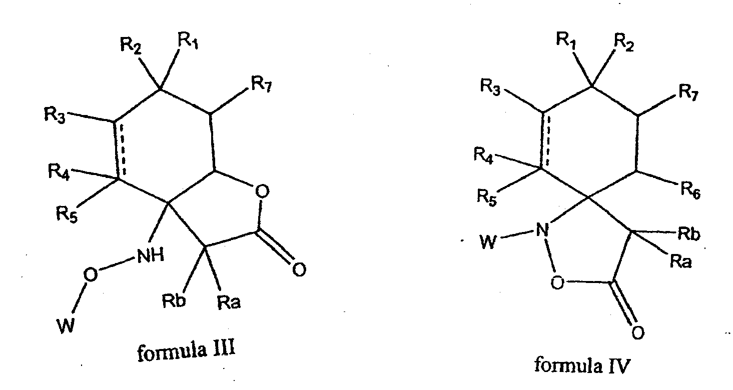

## Description

### FIELD OF THE INVENTION

The present invention refers to a process for rearranging a spirolactam to yield an oxabicyclo[4.3.0]none or a 1-aza-2-oxaspiro derivative. The present invention also refers said oxabicyclo[4.3.0]none and 1-aza-2-oxaspiro derivatives.

### BACKGROUND OF THE INVENTION

Tetrodotoxin (TTX), Octahydro-12-(hydroxymethyl)-2-imino-5,9:7,10a-dimethano-10aH-[1,3]dioxocino[6,5-d]pyrimidine-4,7,10,11,12-pentol, is a potent neurotoxin which selectively binds to sodium channel proteins inhibiting its function in the cell membrane. Tetrodotoxin was first isolated in 1909 from the ovaries of the puffer fish, and named after the puffer fish family "Tetraodontidae". Despite being a small molecule, it has an extremely complex structure characterised by a dioxaadamantane skeleton, a guanidine residue at C2 which is part of a hemiaminal at C4, and an orthoacid bridge at C10. As a result, TTX has 4 quaternary carbon atoms and 8 sterogenic centers.

It should also be noted that TTX is usually present as a mixture of two possible tautomers: an ortoester and a hydroxyl lactone. The ratio of both tautomers depends on the media in which TTX is present.

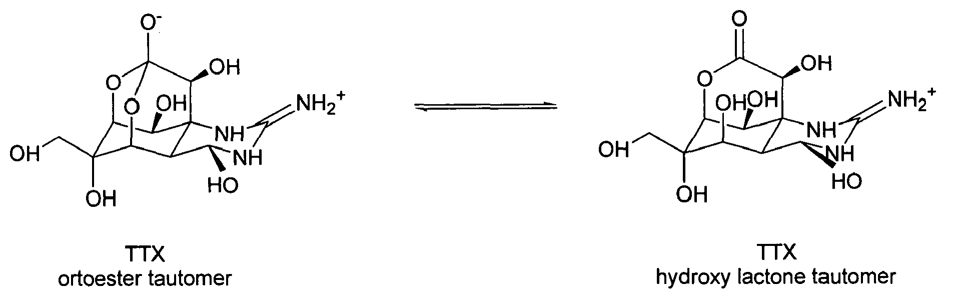

Tetrodotoxin blocks diffusion of sodium through the voltage dependent sodium channel, preventing depolarization and propagation of action potentials in nerve cells. The TTX-Na Channel binding site is extremely tight (*K_{d}* = 10⁻¹⁰ nM). Therefore it is an essential tool in pharmacological studies related to sodium channel proteins.

Further, Tetrodotoxin, due to its analgesic properties, is a promising new drug candidate in the field of pain management. It is currently in phase III clinical trials for cancer pain.

Extraction and purification of natural TTX is complicated, and dependent on the availability of the right animal source. Therefore there is an existing need of providing larger amounts of Tetrodotoxin and its analogues, and researchers seek new cost effective and efficient synthesis. Up to the date, only a few total synthesis have been reported.

The first total synthesis of Tetrodotoxin was reported by Kishi in 1972 and afforded Tetrodotoxin as a racemic mixture after 29 steps in a 0.66% overall yield (Kishi, Y.; Nakatsubo, F.; Aratani, M.; Goto, T.; Inoue, S.; Kakoi, H.; Tetrahedron Lett. 1970, 59, 5127-5128; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9217-9219; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9219-9220; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9220-9221).

The first asymmetric synthesis of Tetrodotoxin was reported by Isobe *et al.* in January 2003 (Isobe,M. et al, J.Am.Chem.Soc, 2003, 125, 8798-8805). This synthesis relied in a Claissen rearrangement, a Shonogashira coupling and an intramolecular carbamate-ester conjugate addition as key steps. After 67 steps Tetrodotoxin was obtained in a 1,22% overall yield.

Shortly after (June, 2003) Dubois *et al.* reported a second asymmetric synthesis of Tetrodotoxin having a Rhodium-catalazed carbene and nitrene insertion as key step with an overall yield of 0.49% after 32 steps (Du Bois, J.; Hinman, A. J. Am. Chem. Soc. 2003, 125, 11510-11511).

Recently, Isobe *et al.* have reported an additional asymmetric total synthesis comprising 62 steps and an overall yield of approximately 1% from a vinilic methyl intermediate (Isobe, M.; Urabe, D.; Nishikawa, T. Angew. Chem. Int. Ed. 2004, 43, 4782-4785).

Also, Isobe has reported the synthesis of different unnatural analogues of Tetrodotoxin following similar strategies as those reported for Tetrodotoxin. Concretely, Isobe *et al.* has reported the synthesis of (-)-5,11-Dideoxytetrodotoxin (Isobe, M.; Asai, M.; Ohyabu, N.; Yamamoto, N.; Nishikawa, T. Angew. Chem. Int. Ed. 1999, 38, 3081-3084), (-)-8,11-Dideoxytetrodotoxin (Isobe, M.; Asai, M.; Iwabuchi, T.; Yoshida, K.; Urabe, D.; Nishikawa, T. Org. Lett. 2002, 16, 2679-2682; Isobe, M.; Asai, M.; Iwabuchi, T.; Yoshida, K.; Urabe, D.; Nishikawa, T. Chem. Eur. J. 2004, 10, 452-462) and 11-Deoxytetrodotoxin (Isobe, M.; Asai, Nishikawa, T. J. Am. Chem. Soc. 2002, 124, 7847-7852).

Sato *et al.* have recently reported an asymmetric total synthesis of Tetrodotoxin from myo-inositol in approximately 0.14% overall yield after 40 steps (Sato, K.; Akai, S.; Sugita, N.; Ohsawa, T.; Kogure, T.; Shoji, H.; Yoshimura, J. J. Org. Chem. 2005, 70, 7496-7504).

Also, further discussions regarding different synthetic approaches to Tetrodotoxin and its analogues may be reviewed in Koert, U. T. Angew. Chem. Int. Ed. 2004, 43, 5572-55769 .

Our copending patent application EP05077580.8 discloses a method for the preparation of TTX and derivate thereof. Said synthesis comprises the rearrangement of a compound having the formula V to yield a compound of formula VI

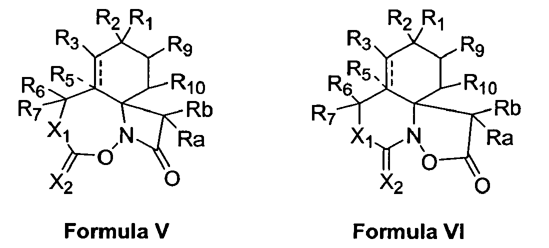

In view of all of the above there is an existing need of providing an alternative cost effective and efficient synthesis of Tetradotoxin and analogues thereof, as well as intermediates therefor, which can be used for the industrial production of sufficient amounts of these compounds. Further, it will open the way to new analogues with useful biological and pharmacological properties.

### SUMMARY OF THE INVENTION

The authors have found that the rearrangement of a spirolactam of formula I as described below in the presence of an acid reagent, yields compounds of formula II, III or IV (further described bellow), which are useful intermediates in the synthesis of TTX and analogues and derivatives thereof.
Thus, a first aspect of the present invention is a process for rearranging the spirolactam of a compound of formula I 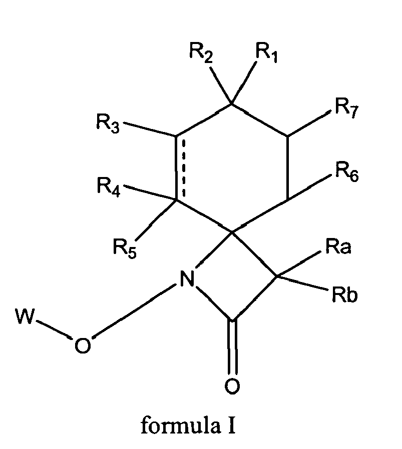 wherein
R₁ is selected from the group consisting of-H, -OH and -OPr₁;
R₂ is selected from the group consisting of -H, -CH₂OH and -CH₂OPr₂; or
R₁ and R₂ together are =O, substituted or unsubstituted alkenyl, -CH₂-O-, or -O-Pr_{cy}-O-CH₂-;
R₃ is selected from the group consisting of -H, -OH, -OPr₃ and =O;
R₄ is selected from the group consisting of -H, cyano, substituted or unsubstituted alkyl, -OPrl₄, -OPr₄, -OH, substituted or unsubstituted alkenyl and substituted or unsubstituted alkynyl;
the dotted lines represent an optional additional bond;
if the additional bond is present then R₅ is not present;
if the bond between C8 and C9 is a single bond, R₅ is selected from the group consisting of -H, -OH and -OPr₅; or
R₄ and R₅ together are =O; or
R₃ and R₅ or R₃ and R₂ together are -O-, forming an epoxide ring;
R₆ is selected from -H, -OH, -OPrl₆, -OPr₆ and =O;
R₇ is selected from the group consisting of -H, -OH, -OPr₇ and =O; or
R₇ and R₆ together are -O-Pr_{cy}-O-;
Ra and Rb are each independently selected from the group consisting of -H, -OH, -OPrab, substituted or unsubstituted alkyl, substituted or unsubstituted amino and halogen;
Pr₁, Pr₂, Pr₃, Pr₄, Pr₅, Pr₆, Pr₇, Pr_{cy} and Prab, are, the same or different, hydroxyl protecting groups;
Prl₆ and Prl₄ are acid labile hydroxyl protecting groups;
W is selected from the group consisting of -H, substituted or unsubstituted alkyl, substituted or unsubstituted aryl, substituted or unsubstituted aralkyl, substituted or unsubstituted heterocyclyl and substituted or unsubstituted alkenyl;
or its tautomers, salts or solvates thereof;
wherein
A) if R₄ is -OH or -OPrl₄ and the compound of formula I is reacted with an acid reagent, a compound of formula II is formed 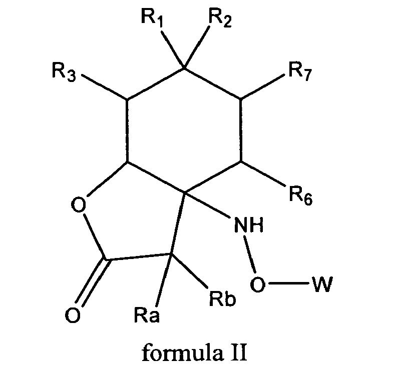 wherein R₁, R₂, R₃, R₆, R₇, Ra, Rb and W are as defined in formula I; or its tautomers, salts or solvates thereof; or
B) if R₆ is -OH or -OPrl₆ and the compound of formula I is reacted with an acid reagent, a compound of formula III is formed 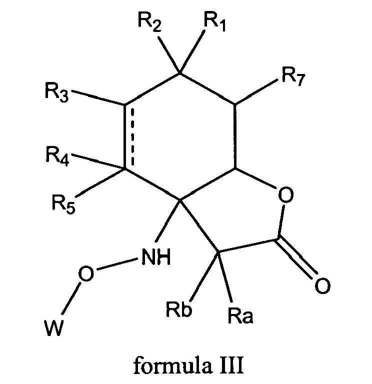 wherein R₁, R₂, R₃, R₄, R₅, R₇, Ra, Rb and W are as defined in formula I; or its tautomers, salts or solvates thereof; or
C) if R₄ is not -OH or -OPrl₄, and R₆ is not -OH or -OPrl₆, and the compound of formula I is reacted with an acid reagent or with halogen hydride, a compound of formula IV is formed 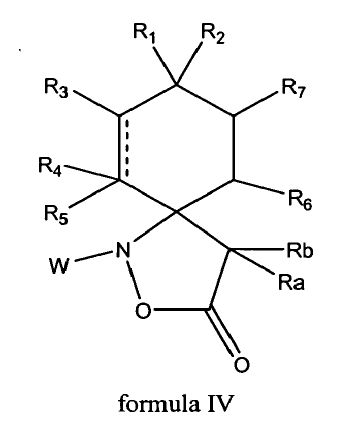 wherein R₁, R₂, R₃, R₄, R₅, R₆, R₇, Ra, Rb and W are as defined in formula I; or its tautomers, salts or solvates thereof

A further aspect of the invention is a compound of formula II as defined in above or its tautomers, salts or solvates thereof.

A further aspect of the invention is a compound of formula III as defined in above or its tautomers, salts or solvates thereof.

A further aspect of the invention is a compound of formula IV as defined in above or its tautomers, salts or solvates thereof.

### DETAILED DESCRIPTION OF THE INVENTION

### Synthesis of compounds of formula II, III and IV

In their ongoing research effort to provide alternative methods for the synthesis of TTX and derivates thereof, the authors found that, surprisingly, when either of R₄ or R₆ in a compound of formula I are deprotected hydroxyl groups or protected hydroxyl groups with protective groups labile in acid media, deprotection of the hydroxyl group is followed by cyclation in the same reaction media to efficiently yield the compounds of formula II or III, their tautomers, salts or solvates thereof.

Further, in the absence of deprotected hydroxyl groups or protected hydroxyl groups with acid labile protecting groups in R₄ and R₆, the reaction follows an alternative route to yield a compound of formula IV. The same compound of formula IV, its tautomers, salts or solvates thereof, may be obtained be reacting the compound of formula I with an halogen hydride.

Thus, as mentioned in the summary of the invention, a first aspect of the present invention is a process for rearranging the spirolactam of a compound of formula I as defined above to yield compounds of formula II, III or IV, its tautomers, salts or solvates thereof.

For purposes of the present invention, the compounds of general formula I, II, III and IV have been numbered as follows:

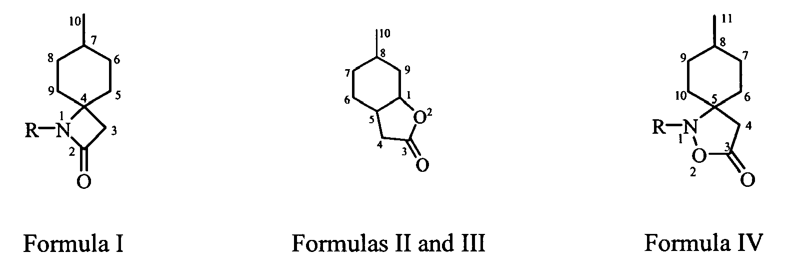

The compound of formula I used as starting material may be readily obtained by the processes described in our copending PCT Patent Applications PCT/EP2005/005146 and PCT/EP2005/005149 and European Patent Applications EP04380295.8 and EP05077580.8, which are herein incorporated by reference in their entirety.

The compounds of formula II, III and IV or their tautomers, salts or solvates thereof, obtained in the process of the invention are useful intermediates in the synthesis of TTX and derivatives thereof. As mentioned above, the process of the invention takes alternative routes depending on the nature of R₄ and R₆. Thus, according to an embodiment, the process of the invention comprises reacting said compound of formula I, or its tautomers, salts or solvates thereof, wherein R₄ is selected from the group consisting of -OH and -OPrl₄, and R₁, R₂, R₃, R₅, R₆, R₇, Ra, Rb and W are as previously defined, with an acid reagent to yield said compound of formula II or its tautomers, salts or solvates thereof.

According to a further embodiment, the process of the invention comprises reacting said compound of formula I, or its tautomers, salts or solvates thereof, wherein R₆ is selected from the group consisting of -OH and -OPrl₆, and R₁, R₂, R₃, R₄, R₅, R₇, Ra, Rb and W are as previously defined, with an acid reagent to yield said compound of formula III or their tautomers, salts or solvates thereof

According to a further embodiment, the process of the invention comprises reacting said compound of formula I, or their tautomers, salts or solvates thereof, wherein R₄ is not -OH or -OPrl₄, R₆ is not -OH or -OPrl₆, and R₁, R₂, R₃, R₅, R₇, Ra, Rb and W are as previously defined, with an acid reagent or with a halogen hydride to yield said compound of formula IV, or their tautomers, salts or solvates thereof.

In order to obtain the compounds of formula II or III it is necessary that either R₄ or R₆ are deprotected hydroxyl groups or protected hydroxyl groups with groups labile in acid media. The skilled person is aware of the protecting groups which are readily cleaved under acid conditions. For further reference of hydroxyl protecting groups which are cleaved under acid conditions, see the reactivity charts present in pages 705-720 of Greene and Wuts' "Protective Groups in Organic Synthesis", John Wiley & Sons, Inc., New York, 3rd Edition, 1999. According to a preferred embodiment, Prl₆ and Prl₄ are independently selected from the group consisting of silyl ethers, preferably silyl ethers of formula -Si(R')₃, such as trimethylsilyl ether (also represented as "TMS"), triethylsilyl ether, tert-butyldimethylsilyl ether (also represented as "TBDMSO"), tert-butyldiphenylsilyl ether, tri-isopropylsilyl ether, diethylisopropylsilyl ether, thexyldimethylsilyl ether, triphenylsilyl ether, di-tert-butylmethylsilyl ether; or esters of formula -C(=O)R', such as acetate ester, benzoate ester; pivalate ester; chloroacetate ester; levulinate ester; wherein R' represents a substituent selected from the group consisting of substituted of unsubstituted alkyl, substituted of unsubstituted alkenyl, substituted of unsubstituted alkynyl, substituted of unsubstituted aryl and substituted of unsubstituted aralkyl

The reaction conditions and the acid reagent to be used for process of the invention depend on whether R₄ or R₆ are protected or deprotected. If either of R₄ or R₆ is protected, the nature of the protecting group must also be considered. According to a preferred embodiment, said acid reagent is selected form the group consisting of silyl triflates (also represented as "Tf"), preferably TMSOTf , and diluted acids, preferably diluted sulphuric acid, diluted hydrochloric acid or diluted acetic acid. "Diluted acid" makes reference to an acid with a concentration in solution bellow 20% wt, preferably bellow 10% wt, more preferably bellow 5% wt.

According to a preferred embodiment, said halogen hydride is sodium hydride. Further, in the synthesis of complex natural products it is always advantageous to perform more than one reaction in a single synthetic step. The skilled person is aware that a hydroxyl group reacts with hydrides to form hydrogen gas and the corresponding alcoxide having a negative charge. Said alcoxide may further react with an electrophile. In fact, this is a common method used to introduce protecting groups in hydroxyl moieties. Thus, according to a preferred embodiment, if any of R₁, R₂, R₃, R₅, R₇, Ra or Rb comprise a hydroxyl group, the process of the invention comprises reacting the compound of formula I, its tautomers, salts or solvates thereof, with a halogen hydride and an electrophile. In this way it is possible to simultaneously rearrange the spirolactam moiety and protect one or more hydroxyl groups of the compound of formula I. There are a number of electrophiles known in the art which are suitable. Reference is made to Greene and Wuts' "Protective Groups in Organic Synthesis", John Wiley & Sons, Inc., New York, 1999. Electrophiles commonly used are halides of formula R₈X, wherein X is a halogen atom, and R₈ is selected from the group consisting of substituted or unsubstituted alkyl, substituted or unsubstituted alkenyl, substituted or unsubstituted aryl, substituted or unsubstituted aralkyl, substituted or unsubstituted heterocyclyl, -C(O)R₉, -C(O)OR₉ and -C(O)NR₉R₁₀, wherein each of R₉ and R₁₀ is independently selected from the group consisting of substituted or unsubstituted alkyl, substituted or unsubstituted alkenyl, substituted or unsubstituted aryl, substituted or unsubstituted aralkyl and substituted or unsubstituted heterocyclyl.

The compounds of formula I, II, III and IV or their tautomers, salts or solvates thereof, may have vecinal hydroxyl groups, allowing the simultaneous protection of two of them through the use of diol protecting groups if desired. Among the diol protecting groups that can be used we have O,O-acetals such as isopropylidene acetals (acetonides); cyclohexylidene and cyclopentylidene acetals; arylmethylene acetals; methylene acetals; diphenylmethylene acetals; 1,2-diacetals such as dispiroketal (dispoke) derivatives, cyclohexane-1,2-diacetals, butane-2,3-diacetals; silylene derivatives; 1,1,3,3-tetraisopropyldisiloxanylidene derivatives or N,O-acetals. Additional examples of diol protecting groups can be found in reference books such as Greene and Wuts' "Protective Groups in Organic Synthesis", John Wiley & Sons, Inc., New York, 1999. Additionally, borolanes can be formed on the two vicinal hydroxy groups, for example using phenylboric acid. The use of protecting groups of a certain size, or that are linked simultaneously to two different positions can control the stereochemistry of the product.

Further, it will be immediately apparent to the skilled person that a compound of formula I or its tautomers, salts or solvates thereof, can be readily transformed into a compound having the same formula I through known reactions. The same is true for the compounds of formula II, III and IV their tautomers, salts or solvates thereof. That is, a compound of formula II can be further transformed into a compound having the same formula II or its tautomers, salts or solvates thereof, a compound of formula III can be further transformed into a compound having the same formula III or its tautomers, salts or solvates thereof, and a compound of formula IV can be further transformed into a compound having the same formula IV or its tautomers, salts or solvates thereof. Said transformations are for example disclosed in our copending applications mentioned above, and may be effected by applying a basic set of reactions selected from:
a) Hydroxilation, dihydroxylation or ketohydroxylation: using mild (such as OsO₄/ N oxide amine) or strong systems (such as RuCl₃) depending on the position to be hydroxylated. Alternative systems are also envisaged.
b) Nucleophilic attack at the carbonyl group: for example with a carbanion on a sp, sp2 or sp3 C, the carbanion can be prepared or generated in situ; or with an hydride. The Nucleophilic attack allows the introduction of new functionalities, new carbonated structures and also epoxides or double bonds, depending on the reagents used.
c) Hydroxyl inversion : for example through epimerization or inversion, for example in Mitsunobu conditions.
d) Hydroxyl or carbonyl protection or deprotection: using the same or different protecting groups in conditions as explained above.
e) Allylic rearrangements: allows migration of a double bond.

For example, a compound of formula I wherein R₄ and R₅ together are =O, may undergo triflation followed by nucleophilic attack by cyanide. Alternatively, said carbonyl in C9 may be directly attacked by cyanide, optionally followed by dehydration. The resulting compounds may be further used to obtain compounds of formula III or IV following the process of the invention:

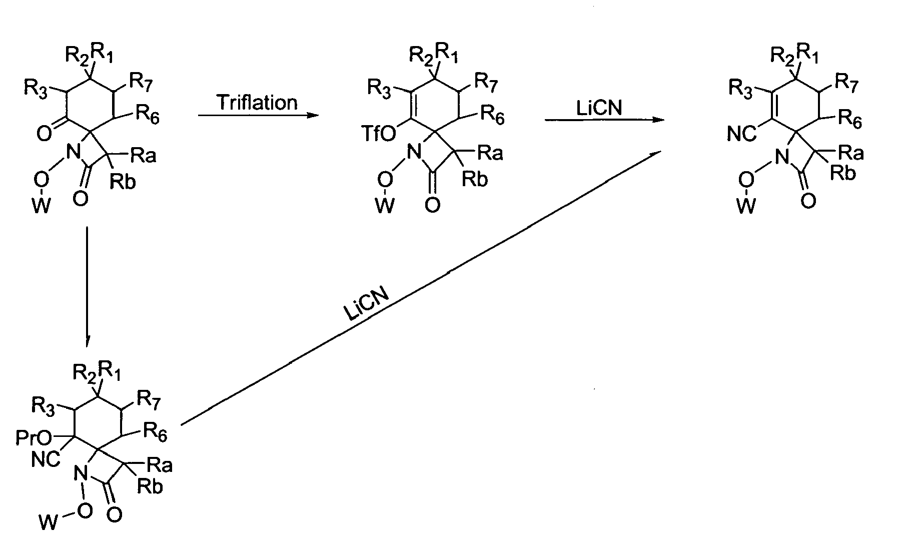

In our previous patent applications EP 04076477.1 and PCT/EP2005/005149 we devised different reaction sequences in order to arrive to the correct structure of R₁ and R₂. The same reaction sequences can also be successfully applied to compounds of formula I, II, III and IV of the invention. For example, introduction of C11 of TTX in a compound of formula IV wherein R₁ and R₂ together are =O, can be performed in three different ways:

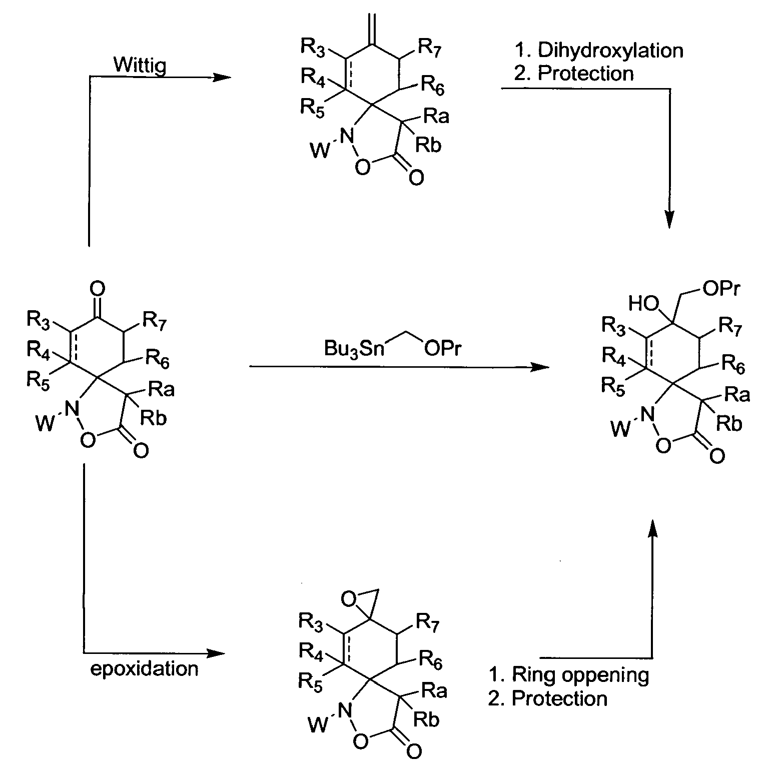

Also, double bonds present in any of the compounds of formula I, II, III or IV may undergo reactions such as epoxidation, dihydroxylation or hydrogenation:

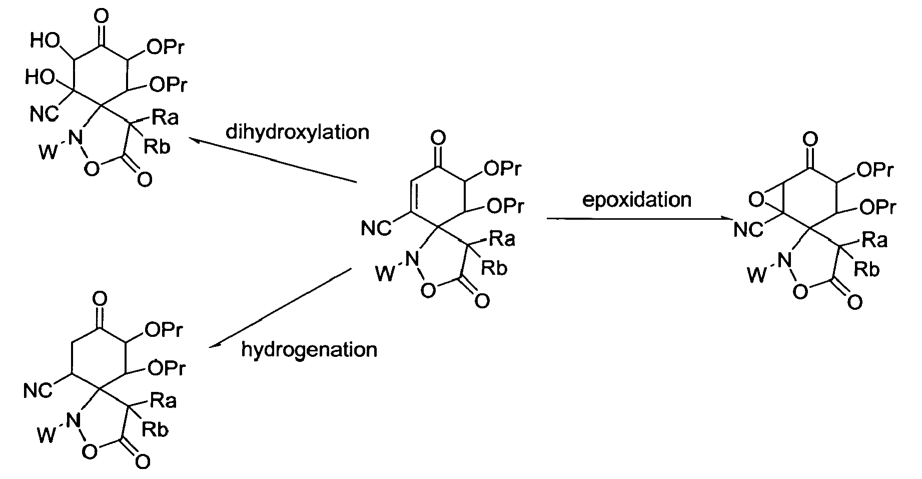

During the total synthesis of organic compounds it is some times necessary to change a protecting group at a given position for different reasons. The present invention encompasses such transformations. The skilled person is aware of the conditions under which it is necessary to exchange the hydroxyl protecting group or an amino protecting group at a given time.

One of the characteristics of TTX is the presence of a guanidine moiety. Our co-pending patent application EP05077580.8, discloses the possibility of introducing the guanidine moiety at an early stage in the synthesis. The same strategy is also possible with the compounds of the present invention. Thus, any of the compounds of formula II, III or IV may undergo a nitrogen deprotection reaction followed by reaction with a guanidine precursor:

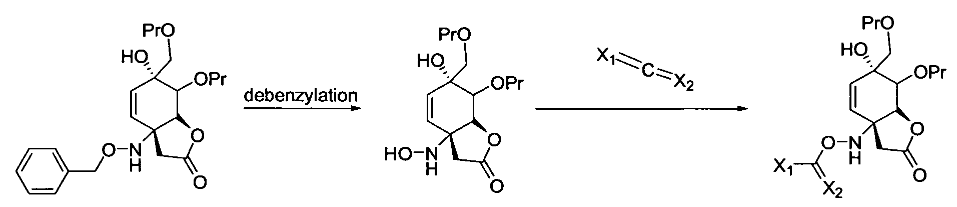

Wherein, X₁ and X₂ are independently selected from NH, O or S. Any of the hydrogen atoms attached to the nitrogen atom in X₁ or X₂, may be replaced by an amino protecting as disclosed in our co-pending patent application EP05077580.8. Alternatively, the guanidine precursor may be a compound of formula 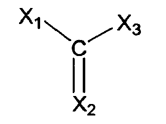 wherein X₂ has the same meaning as above; and X₁ and X₃ are independently selected from the group consisting of -NH₂, -OH, alkoxy, -OPr, -SH and -Salkyl. Any of the hydrogen atoms attached to the nitrogen atom in X₁, X₂ or X₃, may be replaced by an amino protecting as disclosed in our co-pending patent application EP05077580.8.

Additional examples of amino protecting groups can be found in reference books such as Greene and Wuts' "Protective Groups in Organic Synthesis", John Wiley & Sons, Inc., New York, 1999.

A further feature of TTX is the presence of a hydroxyl group at C9, which may be introduced using conditions known to the skilled person. For example, formation of the corresponding enolate followed by addition of oxaziridine to a compound of formula I, II, III or IV results is the introduction of the hydroxyl group (see J. Org. Chem. 1992, 57,6387-6389). The hydroxyl group can then be protected with any desired hydroxyl protecting group.

The introduction of the hydroxyl group at C9 results in two possible diastereoisomers. Thus, the compounds of formula I, II, III or IV having the opposite configuration at C9 with regard to TTX can be obtained as the mayor product. These derivatives are also useful, since epimerization of different hydroxyl functional groups, including C9, throughout the synthesis can be carried out under Mitsonobu conditions (Mitsunobu, 0.; Synthesis, 1, 1981)

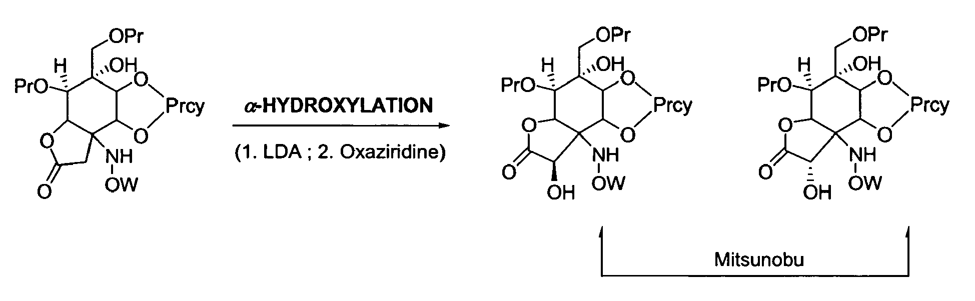

### Compounds of formula II, III and IV

A further aspect of the invention is a compound of formula II as defined above or its tautomers, salts or solvates thereof.

In order to obtain TTX and its derivatives, the compound of formula II, its tautomers, salts or solvates thereof, is preferably one wherein R₃ is -OH or -OPr₃. According to a further embodiment R₇ is -OH or -OPr₇.

As mentioned above, in the synthesis of complex natural products it is always advantageous to perform more than one reaction in a single synthetic step. Besides R₄ or R₆, either of R₁, R₂, R₃, R₅, R₇, Ra or Rb may also be hydroxyl protected groups. According to the process of the invention, the compound of formula I may be treated with an acid reagent. If any of R₁, R₂, R₃, R₅, R₇, Ra or Rb are a hydroxyl group protected, said hydroxyl protecting group will be also cleaved if it is an acid labile hydroxyl protecting group. Thus, according to a preferred embodiment, at least one of Pr₁, Pr₂, Pr₃, Pr₅, Pr₇, Prcy, and Prab is an acid labile hydroxyl protecting group, preferably Pr₃ or Pr₇.

According to an embodiment, R₇ and R₆ together are -0-Prcy-O-.

According to a preferred embodiment, W in -CH₂-Ph.

According to a preferred embodiment, the compound of formula II is a compound of formula IIb 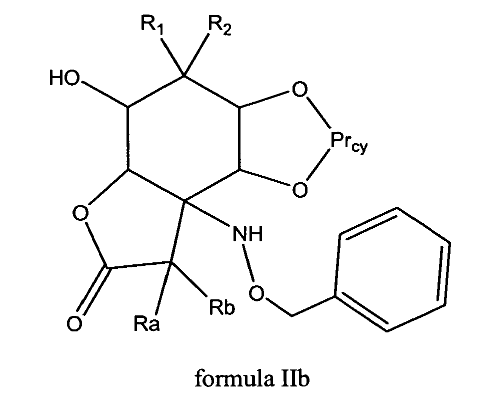 wherein R₁, R₂,Ra, Rb and Pr_{cy} are as defined above;
or its tautomers, salts or solvates thereof.

According to a preferred embodiment, the compound of formula II is selected from
- *rac*-(1*R,*5*R,*6*S,*7*S,*8*S,*9*R*)-5-(benzyloxyamino)-8-(benzoyloxymethyl)-9-(*tert*-butyldimethylsilyloxy)-6,7,8-trihydroxy-6,7-*O*-(1,1,3,3-tetraisopropyldisiloxano-1,3-diyl)-2-oxabicyclo[4.3.0]nonan-3-one: 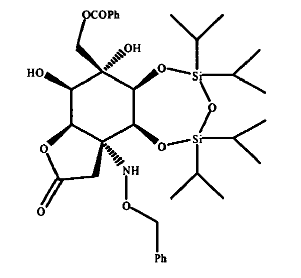 or its enantiomers, tautomers, salts or solvates thereof; or
- *rac*-(*1R,5R,6S,7S,8S,9R*)-5-(benzyloxyamino)-8-(benzoyloxymethyl) - 6,7,8,9-tetrahydroxy -6,7-O-(1,1,3,3-tetraisopropyldisiloxane-1,3-diyl)-2- oxabicyclo[4.3.0]nonan-3-one: 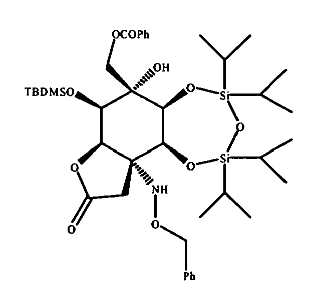 or its enantiomers, tautomers, salts or solvates thereof.

A further aspect of the invention is a compound of formula III as defined above or its tautomers, salts or solvates thereof. According to a preferred embodiment, R₃ is -OH or -OPr₃. According to a further embodiment, R₇ is -OH or -OPr₇. According to a further embodiment, W in -CH₂-Ph.

According to a preferred embodiment, at least one of Pr₁, Pr₂, Pr₃, Pr₅, Pr₇, Prcy and Prab is an acid labile hydroxyl protecting group, preferably Pr₃ or Pr₇. According to a preferred embodiment the compound of formula III is a compound of formula IIIb 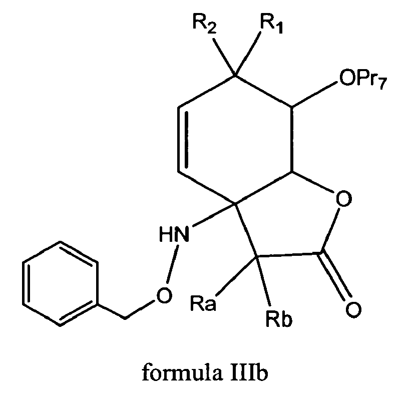 wherein R₁, R₂, Ra, Rb and Pr₇ are as defined above;
or its tautomers, salts or solvates thereof

According to a preferred embodiment, the compound of formula III is selected from the group consisting of
- rac-(1S,5R,9S)-5-(benzyloxyamino)-9-hydroxy-2-oxabicyclo[4.3.0]non-6-en-3,8-dione: 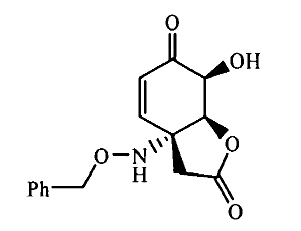 or its enantiomers, tautomers, salts or solvates thereof;
- rac-(1S,5R,9S)-5-(benzyloxyamino)-9-(tert-butyldimethylsilyloxy)-2-oxabicyclo[4.3.0]non-6-en-3,8-dione: 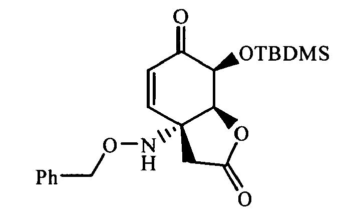 or its enantiomers, tautomers, salts or solvates thereof;
- rac-(1S,5R,9S)-5-(benzyloxyamino)-9-(tert-butyldimethylsilyloxy)-8-methylen-2-oxabicyclo[4.3.0]non-6-en-3-one: 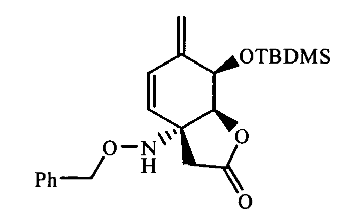 or its enantiomers, tautomers, salts or solvates thereof;
- rac-(1S,5R,9S)-5-(benzyloxyamino)-9-(tert-butyldimethylsilyloxy)-8-hydroxy-8-hydroxymethyl-2-oxabicyclo[4.3.0]non-6-en-3-one: 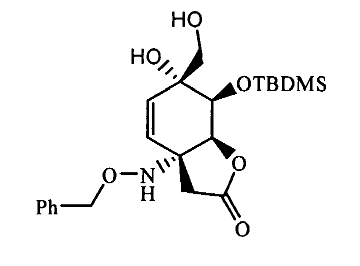 or its enantiomers, tautomers, salts or solvates thereof; and
- rac-(1S,5R,9S)-5-(benzyloxyamino)-9-(tert-butyldimethylsilyloxy)-8-hydroxy-8-(tert-butyldimethylsilyloxymethyl)-2-oxabicyclo[4.3.0]non-6-en-3-one: 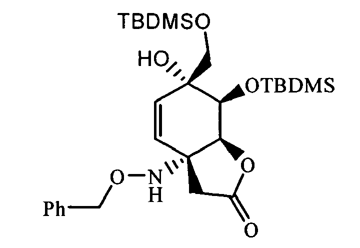 or its enantiomers, tautomers, salts or solvates thereof.

A further aspect of the present invention is a compound of formula IV as defined above or its tautomers, salts or solvates thereof.

According to a preferred embodiment, the compound of formula IV is a compound of formula IVb 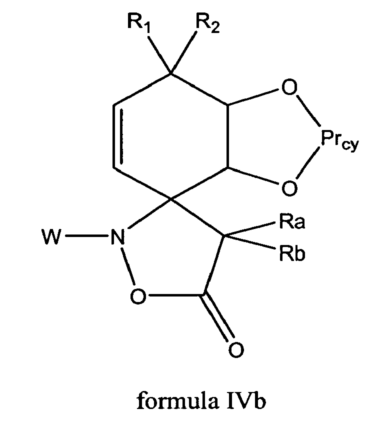 wherein R₁, R₂, Pr_{cy} and W are as defined above;
or its enantiomers, tautomers, salts or solvates thereof.

According to a preferred embodiment, the compound of formula IV is selected from the group consisting of
- rac-(5R,6S,7S,8R)-1-benzyl-6,7,8-trihydroxy-8-[(4-methoxybenzyloxy)methyl] -6,7-O-(1,1,3,3-tetraisopropyldisiloxane-1,3-diyl)-1-aza-2-oxaspiro[4.5]dec-9-en-3-one: 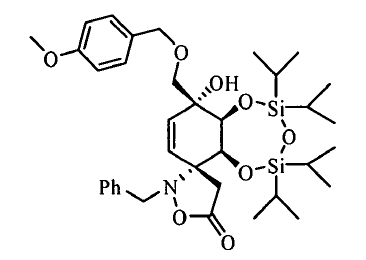 or its enantiomers, tautomers, salts or solvates thereof;
- rac-(5R,6S,75,8R)-1-benzyl-6,7,8-trihydroxy-8-(hydroxymethyl)-6,7-O-(1,1,3,3-tetraisopropyldisiloxane-1,3-diyl)-1-aza-2-oxaspiro[4.5]dec-9-en-3-one: 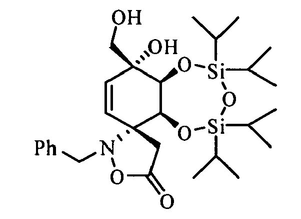 or its enantiomers, tautomers, salts or solvates thereof;
- rac-(5*R*,6*S*,7*S*)-1-benzyl-6,7-dihydroxy-6,7-O-(1,1,3,3,-tetraisopropyl disiloxane-1,3-diyl)-1-aza-2-oxaspiro[4.5]dec-9-en-3,8-dione: 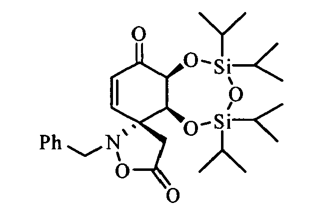 or its enantiomers, tautomers, salts or solvates thereof; and
- rac-(5R,6S,7S)-6,7-dihydroxy-6,7-O-(1,1,3,3,-tetraisopropyldisiloxane-1,3-diyl)-1-aza-2-oxaspiro[4.5]dec-9-en-3,8-dione: 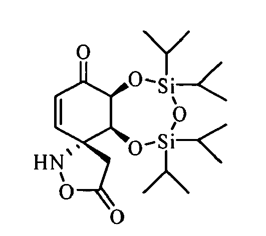 or its enantiomers, tautomers, salts or solvates thereof

### Definitions

In the definition of compounds disclosed herein and in the description, the following terms have the meaning indicated:
**"Alkyl"** refers to a straight or branched hydrocarbon chain radical consisting of carbon and hydrogen atoms, containing no saturation, having 1-12, preferably one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as halo, hydroxy, alkoxy, OPr, OBn, OBz, carboxy, cyano, carbonyl, acyl, alkoxycarbonyl, amino, imino, nitro, mercapto and alkylthio.
**"Alkenyl"** refers to a straight or branched hydrocarbon chain radical consisting of carbon and hydrogen atoms, containing at least one unsaturation, having 1-12, preferably one to eight carbon atoms, and which is attached to the rest of the molecule by a single bond or by a double bond, e.g. -CH=CH₂, -CH=CHCH₃, -CH₂-(CH=CH)ₙCH₃, wherein n is an integer between 1 and 5, =CH₂, =CHCH₃. Alkenyl radicals may be optionally substituted by one or more substituents such as halo, hydroxy, alkoxy, O-propyl, O-benzyl, O-benzoate, carboxy, cyano, carbonyl, acyl, alkoxycarbonyl, amino, imino, nitro, mercapto and alkylthio.
**"alkynyl"** refers to a straight or branched hydrocarbon chain radical consisting of carbon and hydrogen atoms, containing at least one carbon-carbon triple bond, conjugated of not, and which is attached to the rest of the molecule by a single bond, such as -CCH, -CH₂CCH, -CCCH₃, -CH₂CCCH₃.
"**Alkoxy**" refers to a radical of the formula -ORa where Ra is an alkyl radical as defined above, e. g., methoxy, ethoxy, propoxy, etc. "Aryloxy" refers to a radical of formula -ORb wherein Rb is an aryl radical as defined below. "Amino" refers to a radical of the formula-NH₂ , -NHRa, -NRaRb.
**"Aryl"** refers to an aromatic hydrocarbon radical such as phenyl (also represented as "Ph"), naphthyl or anthracyl. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
**"Aralkyl"** refers to an aryl group linked to an alkyl group such as benzyl and phenethyl.
**"Heterocyclyl"** refers to a stable 3- to 15- membered ring which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.
**"Amino"** refers to a radical of the formula -NH₂, -NHRa, -NRaRb, wherein Ra and Rb are independently selected from substituted or unsubstituted alkyl, substituted or unsubstituted cycloalkyl, substituted or unsubstituted alkenyl, substituted or unsubstituted cycloalkenyl, substituted or unsubstituted aryl, substituted or unsubstituted aralkyl or substituted or unsubstituted heterocyclyl; or Ra and Rb together form a substituted or unsubstituted heterocycle, substituted or unsubstituted cycloalkyl or substituted or unsubstituted cycloalkenyl.
**"halogen"** refers to -F, -Cl, -Br or -I.
**"Protecting group"** refers to a group that blocks an organic functional group and can be removed under controlled conditions. Protecting groups, their relative reactivity and conditions under which they remain inert are known to the skilled person. Reference is made to Greene and Wuts "Protective Groups in Organic Synthesis", John Wiley & Sons, Inc., New York, 1999.
**"Hydroxyl protecting group"** refers to a group that blocks the -OH function for further reactions and can be removed under controlled conditions. The hydroxyl protecting groups are well known in the art, representative protecting groups are:
   - silyl ethers of formula -Si(R')₃, such as trimethylsilyl ether (also represented as "TMS"), triethylsilyl ether, tert-butyldimethylsilyl ether (also represented as "TBDMSO"), tert-butyldiphenylsilyl ether, tri-isopropylsilyl ether, diethylisopropylsilyl ether, thexyldimethylsilyl ether, triphenylsilyl ether, di-tert-butylmethylsilyl ether;
   - alkyl ethers of formula -R', such as methyl ether, tert-butyl ether, benzyl ether, p-methoxybenzyl ether, 3,4-dimethoxybenzyl ether, trityl ether; allyl ether;
   - alkoxymethyl ethers of formula -CH₂-O-R', such as methoxymethyl ether, 2-methoxyethoxymethyl ether, benzyloxymethyl ether, p-methoxybenzyloxymethyl ether, 2-(trimethylsilyl)ethoxymethyl ether. The oxygen atom may be replaced by a sulphur atom to form an alkylthiomethyl ether of formula -CH₂-S-R', such as methylthiomethyl ether. Tetrahydropyranyl and related ethers are also commonly used as hydroxyl protecting groups;
   - esters of formula -C(=O)R', such as acetate ester, benzoate ester; pivalate ester; methoxyacetate ester; chloroacetate ester; levulinate ester;
   - carbonates of formula -C(=O)-O-R', such as benzyl carbonate, p-nitrobenzyl carbonate, tert-butyl carbonate, 2,2,2-trichloroethyl carbonate, 2-(trimethylsilyl)ethyl carbonate, allyl carbonate; or
   - sulphates such as SO_{3.}py.

In all the above formula R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl.

Additional examples of hydroxyl protecting groups can be found in reference books such as Greene and Wuts' "Protective Groups in Organic Synthesis", John Wiley & Sons, Inc., New York, 1999.

References herein to **substituted groups** in the compounds of the present invention refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C₁-C₆ alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; amino groups such as groups having one or more N atoms, optionally substituted with 1 or 2 alkyl groups having from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

All the compounds will be obtained as racemic mixtures. However, if enantiopurity is desired, this can be achieved by introducing a chiral center in the W group, introduncing different groups as Ra and Rb, or using chiral reagents or catalysts. Therefore the compounds of the present invention may include pure enantiomers depending on the presence of stereogenic centers or diastereoisomers. The single isomers, enantiomers or diastereoisomers and mixtures thereof fall within the scope of the present invention. Mixtures of different diasteroisomers can be separated by conventional techniques.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The compounds of the invention may be in crystalline form either as free compounds or as solvates (e.g. hydrates) and it is intended that both forms are within the scope of the present invention. Methods of solvation are generally known within the art.

Further embodiments of the compounds of the invention are described below in the examples. It is evident for the skilled person that many variations and configurations are possible and can be obtained at will depending on the compounds selected as starting materials, the relative configurations of the functional groups present, the presence or not of chiral centers, and on the combination of reactions that are applied. The present invention encompasses all such variations (enantiomers and diastereoisomers) and possibilities.

### EXAMPLES

### General Methods and Materials

All reactions described below were carried out under argon atmosphere unless otherwise noted. The solvents used were distilled and dried under argon atmosphere before use. All starting materials were purchased commercially (Aldrich, Fluka and Merck) and used without further purification. Flash Chromatography was executed on columns loaded with 230-400 mesh silica gel Merck. TLC was carried out on silica gel Merck (Kieselgel 60F-254).

Melting points (mp) were determined on a Reichert Microscopic Hot-Stage and are uncorrected. ¹H and ¹³C NMR spectra were measured on Varian Gemini-200, Varian Inova-300 and Varian Inova-400 spectrometers with (CH₃)₄Si as an internal reference and CDCl₃ as solvent unless otherwise noted. Both ¹H and ¹³C NMR spectral data are reported in parts per million (δ) relative to residual signal of the solvent (CHCl₃, 7.26 ppm and 77.0 ppm for ¹H and ¹³C NMR, respectively). ¹H and ¹³C NMR designations are: s (singlete); br. s (broad singlete); d (doublete); br. d (broad doublete); t (triplete); q (quartete); m (multiplete). Infrared (IR) spectra were record on a Perkin-Elmer FT-IR spectrometer. UV spectra were record on a Perkin-Elmer 402 spectrometer. Low-resolution mass (LRMS) spectra were obtained on a Hewlett Packard 5973 MSD spectrometer with a direct inlet system (EI) at 70 eV.

### Synthesis of compounds of formula II

### Example 1: Preparation of rac-(4R,5S,6S,7S,8R,9R)-1-benzyloxy-7-(benzoyl oxymethyl)-8-(tert-butyldimethylsilyloxy)-5,6,7,9-tetrahydroxy-5,6-O-(1,1,3,3-tetraisopropyldisiloxane-1,3-diyl)-l-azaspiro[3.5]nonan-2-one (15) and rac-(1R,5R,6S,7S,8S,9R)-5-(benzyloxyamino)-8-(benzoyloxymethyl)-9-(tert-butyldimethylsilyloxy)-6,7,8-trihydroxy-6,7-O-(1,1,3,3-tetraisopropyldisiloxano-1,3-diyl)-2-oxabicyclo[4.3.0]nonan-3-one (16)

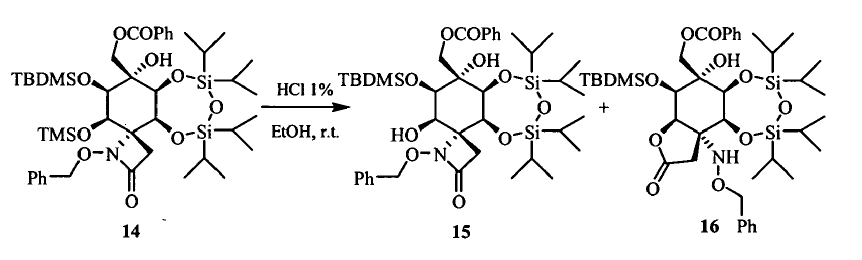

To a solution of *rac*-(4*R*,5*S*,6*S*,7*S*,8*R*,9*R*)-1-benzyloxy-7-(benzoyloxymethyl)-*8-(tert-*butyldimethylsilyloxy)-5,6,7-trihydroxy-5,6-*O*-(1,1,3,3-tetraisopropyldisiloxane-1,3-diyl)-9-(trimethylsilyloxy)-1-azaspiro[3.5]nonan-2-one (14) (293 mg, 0.330 mmol) in 1% HCl (1.8 ml, 1 wt. % solution in EtOH, 0.495 mmol) was stirred at room temperature for 24 h. Then, 1% HCl solution in EtOH (1.8 ml) was added, and the mixture was stirred at room temperature until the reaction was complete (24 h, TLC monitoring, hexane/AcOEt, 2:1). The reaction was neutralized with 10% aqueous NaHCO₃ solution and the mixture extracted with CH₂Cl₂ (3 x 3 ml). The combined extracts were dried over Na₂SO₄, filtered and concentrated under reduced pressure. The residue was purified by silica gel column chromatography (hexane/AcOEt, 5:1) to give *rac*-(4*R*,5*S*,6*S*,7*S*,8*R*,9*R*)-1-benzyloxy-7-(benzoyloxymethyl)-8-(*tert-*butyldimethylsilyloxy)-5,6,7,9-tetrahydroxy-5,6-*O*-(1,1,3,3-tetraisopropyldisiloxane-1,3-diyl)-1-azaspiro[3.5]nonan-2-one (**15**) as a foamy white solid (250 mg, 93%), and *rac*-(1*R*,5*R*,6*S*,7*S*,8*S*,9*R*)-5-(benzyloxyamino)-8-(benzoyloxymethyl)-9-(*tert-*butyldimethylsilyloxy)-6,7,8-trihydroxy-6,7-*O*-(1,1,3,3-tetraisopropyldisiloxano-1,3-diyl)-2-oxabicyclo[4.3.0]nonan-3-one (**16**) (15 mg, 6%), as a white solid.

### rac-(4R,5S,6S,7S,8R,9R)-1-benzyloxy-7-(benzoyloxymethyl)-8-(tert-butyldi methylsilyloxy)-5,6,7,9-tetrahydroxy-5,6-O-(1,1,3,3-tetraisopropyldisiloxane-1,3-diyl)-1-azaspiro[3.5]nonan-2-one (15)

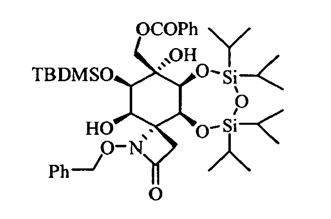

***R_{f}*** = 0.51 (TLC, hexano/AcOEt, 2:1), 0.17 (TLC, hexano/AcOEt, 5:1); **yield,** 93%; white solid; **m.p.** = 87-89 °C; **¹H-NMR** (300 MHz, CDCl₃): δ 8.03 (2H, dm, J = 7.1 Hz, Bz), 7.62 (1H, tm, J = 7.3 Hz, Bz), 7.48 (2H, ddm, J = 7.3, 7.1 Hz, Bz), 7.45-7.35 (5H, m, Ph), 5.07 (1H, *part A syst. AB,* J = 11.4 Hz, OCH₂Ph), 4.99 (1H, *part B syst. AB,* J = 11.4 Hz, OCH₂Ph), 4.67 (1H, *part A syst. AB,* J = 11.6 Hz, H-10), 4.58 (1H, *part B syst. AB,* J = 11.6 Hz, H-10'), 4.52 (1H, d, J = 4.0 Hz, H-5), 4,35 (1H, dd, J = 4.0, 0.9 Hz, H-6), 4.03 (1H, dd, J = 3.4, 0.9 Hz, H-8), 3.86 (1H, dd, J = 5.0, 3.4 Hz, H-9), 2.98 (1H, *part A syst. AB,* J = 14.1 Hz, H-3), 2.89 (1H, *part B syst. AB,* J = 14.1 Hz, H-3'), 2.70 (1H, s, HO-C(7)), 1.62 (1H, d, J = 5.0 Hz, HO-C(9)), 1.14-0.97 (28H, m, TIPDS), 0.87 (9H, s, C(CH₃)₃), 0.12 (3H, s, SiCH₃), 0.07 (3H, s, SiCH₃); **¹³C-NMR** (75 MHz, CDCl₃): δ 168.4, 167.6, 136.2, 133.7, 129.6, 129.2, 128.9, 128.75, 128.7, 128.6, 78.5, 75.3, 74.9, 74.8, 69.8, 68.8, 67.9, 66.1, 34.8, 26.2, 18.5, 17.7, 17.65, 17.5, 17.3, 17.25, 17.2, 17.1, 17.0, 14.2, 14.0, 13.2, 13.0, -3.6, -5.5; **IR** (film): v 3434, 2942, 2894, 2862, 1749, 1725, 1461, 1385, 1364, 1313, 1273, 1158, 1107, 1067, 997, 952, 912, 883, 832, 796, 778 cm⁻¹; **LRMS (API-ES**⁺**):** m/z 1653 (2M+Na)⁺, 1631 (2M+H)⁺, 838 (M+Na)⁺, 816 (M+H)⁺.

### rac-(1R,5R,6S,7S,8S,9R)-5-(benzyloxyamino)-8-(benzoyloxymethyl)-9-(tert-butyldimethylsilyloxy)-6,7,8-trihydroxy-6,7-O-(1,1,3,3-tetraisopropyldisiloxano-1,3-diyl)-2-oxabicyclo[4.3.0]nonan-3-one (16)

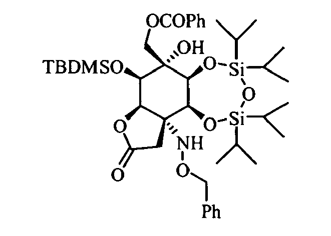

***R_{f}*** = 0.36 (TLC, hexano/AcOEt, 5:1), 0.64 (TLC, hexano/AcOEt, 2:1); **yield,** 6%; white solid; **¹H-NMR** (300 MHz, CDCl₃): δ 8.02 (2H, dm, J = 7.2 Hz, Bz), 7.63 (1H, tm, J = 7.5 Hz, Bz), 7.48 (2H, ddm, J = 7.5, 7.2 Hz, Bz), 7.40-7.24 (5H, m, Ph), 5:82 (1H, s, NH), 4.98 (1H, d, J = 5.3 Hz, H-1), 4,93 (1H, d, J = 4.6 Hz, H-6), 4.82 (1H, *part A syst. AB,* J = 12.0 Hz, OCH₂Ph), 4.73 (1H*, part B syst. AB,* J = 12.0 Hz, OCH₂Ph), 4.63 (2H, s, H-10 and H-10'), 4.47 (1H, d, J = 4.6 Hz, H-7), 4,24 (1H, d, J = 5.3 Hz, H-9), 3.42 (1H, *part A* syst. *AB,* J = 16.9 Hz, H-4), 2.71 (1H, s, OH), 1.85 (1H, *part B syst. AB,* J = 16.9 Hz, H-4'), 1.20-0.95 (28H, m, TIPDS), 0.91 (9H, s, C(CH₃)₃), 0.21 (3H, s, SiCH₃), 0.13 (3H, s, SiCH₃); **¹³C-NMR** (75 MHz, CDCl₃): δ 176.1, 167.3, 137.6, 133.7, 129.6, 129.1, 128.6, 128.3, 127.9, 127.7, 77.2, 76.8, 75.6, 73.5, 71.6, 67.9, 67.8, 67.5, 36.1, 25.9, 18.0, 17.65, 17.6, 17.5, 17.35, 17.3, 17.2, 17.1, 16.7, 13.9, 13.1, 12.7, 11.8, -4.2, -4.8; **IR** (film): ν 3397, 2946, 2890, 2867, 1790 (shoulder), 1760, 1724, 1601, 1463, 1386, 1316, 1273, 1148, 1125, 1099, 1068, 997, 919, 883, 838, 778, 694 cm⁻¹; **LRMS (API-ES⁺):** m/z 862 (M+2Na)⁺, 816 (M+H)⁺; **LRMS (EI):** m/z 758 (M⁺-57, 7), 693 (1), 650 (1), 636 (2), 618 (1), 610 (1), 544 (1), 529 (1), 513 (2), 483 (3), 395 (2), 365 (5), 339 (17), 289 (6), 261 (8), 179 (9), 163 (4), 147 (5), 135 (6), 105 (100), 91 (76), 77 (8).

### Example 2: Preparation of rac-(1R,5R,6S,7S,8S,9R)-5-(benzyloxyamino)-8-(benzoyloxymethyl)-6,7,8,9-tetrahydroxy-6,7-O-(1,1,3,3-tetraisopropyldisiloxane-1,3-diyl)-2-oxabicyclo[4.3.0]nonan-3-one (18)

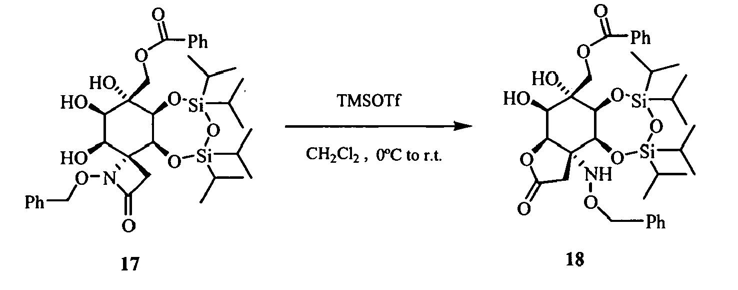

To a stirred solution of *rac*-(4*R*,5*S*,6*S*,7*S*,8*R*,9*R*)-1-benzyloxy-8,9-dihydroxy-5,6-(1,1,3,3-tetraisopropyldisiloxane-1,3-diyl)-10-(benzoyloxymethyl)-1-azaspiro[3.5]nonan-2-one (17) (50 mg, 0.071 mmol) in CH₂Cl₂ (1.5 ml) was added at 0° C trimethylsilyl trifluoromethanesulfonate (16 µl, 0.086 mmol). The resulting mixture was stirred at room temperature until the reaction was completed (1h, TLC monitoring, hexane/AcOEt, 2:1). The reaction was quenched at 0° C with Na₂PO₄ 0.1 M buffer until pH = 7 and the mixture extracted with CH₂Cl₂ (3 x 4 ml). The combined organic extracts were dried over Na₂SO₄, filtered and concentrated under reduced pressure. The residue was purified by silica gel column chromatography (hexane-AcOEt, 3:1) to give *rac*-(1*R,*5*R,*6*S,*7*S,*8*S,*9*R*)-5-(benzyloxyamino)-8-(benzoyloxymethyl)-6,7,8,9-tetrahydroxy-6,7-*O*-(1,1,3,3-tetraisopropyldisiloxane-1,3-diyl)-2-oxabicyclo[4.3.0]nonan-3-one (18) (37 mg, 74%) as a white solid.
***R_{f}* =** 0.37 (TLC, hexane/AcOEt 2:1); **yield**, 74%; white solid; **¹H-NMR** (300 MHz CDCl₃): d 8.04 (2H, d, J = 7.35 Hz, OCOPh), 7.63 (1H, m, OCOPh), 7.48 (2H, m, OCOPh), 7.37-7.28 (5H, m, Ph), 5.72 (1H, br. s, NH), 4.97 (1H, d, J = 4.3 Hz, H-6), 4.93 (1H, d, J = 4.7 Hz, H-1), 4.88 (1H, *part A syst. AB,* J = 12.8 Hz, H-10), 4.81 (1H, *part A syst. AB,* J = 13.2 Hz, OCH₂Ph), 4.73 (1H, *part B syst. AB,* J = 13.2, OCH₂Ph), 4.73 (1H, *part B syst. AB,* J = 12.8 Hz, H-10'), 4.56 (1H, d, J = 4.0 Hz, H-7), 4.20 (1H, dd , J = 8.6, 4.7 Hz, H-9), 3.75 (1H, d, J = 8.6 Hz, OH), 3.28 (1H, br. s, OH), 3.06 (1H, *part A syst. AB,* J = 17.7 Hz, H-4), 1.94 (1H, *part B syst. AB,* J = 17.7 Hz, H-4'), 1.09-0.99 (28H, m, TIPDS); **¹³C-NMR** (100 MHz, CDCl₃): 175.8, 167.9, 137.3, 133.9, 129.8, 128.9, 128.65, 128.58, 128.4, 128.1, 127.8, 78.0, 74.8, 74.0, 71.1, 68.2, 67.8, 97.6, 36.5, 17.52, 17.48, 17.26, 17.21, 17.1, 17.0, 13.9, 13.4, 13.0, 12.7. **IR** (KBr): ? 3462, 3062, 3028, 2946, 2868, 1765, 1725, 1601, 1583, 1494, 1464, 1453, 1385, 1314, 1273, 1145, 1098, 1068, 1000, 885, 755, 698 cm⁻¹; **LMRS (API-ES⁺):** m/z 1425 (2M+Na)⁺, 792 (M+Na+H)⁺, 705 (M+4H)⁺, 704 (M+3H)⁺, 703 (M+2H)⁺, 702 (M+H)⁺.

### Synthesis of compounds of formula III

### Example 3: Preparation of rac-(1S,5R,9S)-5-(benzyloxyamino)-9-hydroxy-2-oxabicyclo[4.3.0]nona-6-en-3,8-dione (12) and/or rac-(1S,5R,9S)-5-(benzyloxyamino)-9-(tert-butyldimethylsilyloxy)-2-oxabicyclo[4.3.0]non-6-en-3,8-dione (13)

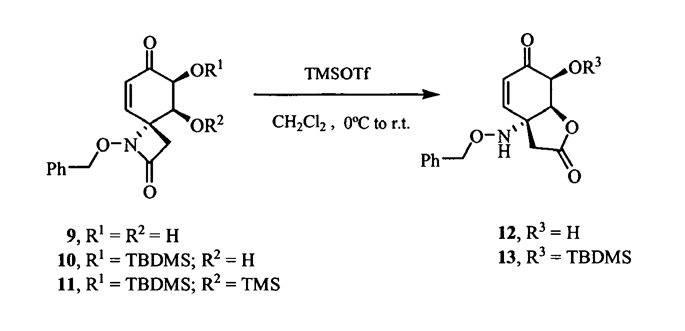

### Procedure A

To a stirred solution of *rac*-(4*R*,5*S*,6*S*)-1-benzyloxy-5,6-dihidroxy-1-azaspiro[3.5]non-8-en-2,7-dione **(9)** (100 mg, 0.346 mmol) in CH₂Cl₂ (3 ml) at 0° C was added trimethylsilyl trifluoromethanesulfonate (76 µl, 0.414 mmol). The resulting mixture was stirred at room temperature until the reaction was complete (4.5 h, TLC monitoring, hexane/AcOEt, 1:5). The reaction was quenched at room temperature with NaHCO₃ solution. The layers were separated and aqueous phase was extracted with CH₂Cl₂ (3 x 4 ml). The combined organic extracts were dried over Na₂SO₄, filtered and concentrated under reduced pressure to give *rac*-(1*S*,5*R*,9*S*)-5-(benzyloxyamino)-9-hydroxy-2-oxabicyclo[4.3.0]non-6-en-3,8-dione **(12)** (20 mg, 20%) as a colourless oil.

### Procedure B

To a stirred solution of *rac*-(4*R*,5*S*,6*S*)-1-benzyloxy-6-*tert*-butyldimethylsilyloxy-5-hydroxy-1-azaspiro[3.5]non-8-en-2,7-dione **(10)** (100 mg, 0.248 mmol) in CH₂Cl₂ (2.5 ml) was added at 0° C trimethylsilyl trifluoromethane-sulfonate (54 µl, 0.297 mmol). The resulting mixture was stirred at room temperature until the reaction was complete (10.5 h, TLC monitoring, hexane/AcOEt, 1:1). The reaction was quenched at 0° C with Na₂PO₄ 0.1 M buffer until pH = 7 and the mixture extracted with CH₂Cl₂ (3 x 4ml). The combined organic extracts were dried over Na₂SO₄, filtered and concentrated under reduced pressure. The residue was purified by silica gel column chromatography (hexane/AcOEt, 3:1) to give *rac-*(1*S*,5*R*,9*S*)-5-(benzyloxyamino)-9-hydroxy-2-oxabicyclo[4.3.0]non-6-en-3,8-dione **(12)** (21 mg, 28%), as a colourless oil, and *rac*-(1*S*,5*R*,9*S*)-5-(benzyloxyamino)-9-(*tert*-butyldimethylsilyloxy)-2-oxabicyclo[4.3.0]non-6-en-3,8-dione (**13**) (70 mg, 70%), as a white solid.

### Procedure C

To a stirred solution of *rac-*(4*R*,5*S*,6*S*)-1-benzyloxy-6-*tert*-butyldimethylsilyloxy-5-trimethylsilyloxy-1-azaspiro[3.5]non-8-en-2,7-dione (11) (100 mg, 0.210 mmol) in CH₂Cl₂ (2 ml) was added at 0° C trimethylsilyl trifluoromethanesulfonate (48 µl, 0.252 mmol). The resulting mixture was stirred at room temperature until the reaction was complete (20 h, TLC monitoring, hexane/AcOEt, 2:1). The reaction was quenched at room temperature with saturated NaHCO₃ solution (1 ml). The layers were separated and aqueous phase was extracted with CH₂Cl₂ (3 x 4 ml). The combined organic extracts were dried over Na₂SO₄, filtered and concentrated under reduced pressure. The residue was purified by silica gel column chromatography (hexane/AcOEt, 4:1) to give *rac-*(1*S,*5*R,*9*S*)-5-(benzyloxyamino)-9-hydroxy-2-oxabicyclo[4.3.0]non-6-en-3,8-dione **(12)** (12 mg, 20%), as a colourless oil, and *rac-*(1*S*,5*R,*9*S*)-5-(benzyloxyamino)-9-(*tert*-butyldimethylsilyloxy)-2-oxabicyclo[4.3.0]non-6-en-3,8-dione **(13)** (44 mg, 52%), as a white solid.

### rac-(1S,5R,9S)-5-(benzyloxyamino)-9-hydroxy-2-oxabicyclo[4.3.0]non-6-en-3,8-dione (12)

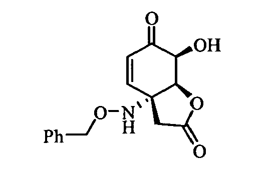

***R_{f}* =** 0.25 (TLC, hexane/AcOEt, 1: 1); **yield,** 20% **(*Procedure A*),** 28% **(*Procedure B*),** 20% **(*Procedure C*);** colourless oil; **¹H-NMR** (300 MHz CDCl₃): d 7.42-7.30 (5H, m, Ph), 6.44 (1H, *part A syst. AB,* J = 10.3, 2.2 Hz, H-6 or H-7), 6.25 (1H, *part B syst. AB,* J = 10.3 Hz, H-7 or H-6), 5.69 (1H, br. s, NH), 4.95 (1H, dd, J=3.4,2.2 Hz,H-1 or H-9), 4.72 (3H, m, OCH₂Ph and H-9 or H-1), 3.47 (1H, br. s, OH), 2.95 (1H, *part A syst. AB,* J = 17.2 Hz, H-4), 2.50 (1H, *part B syst. AB,* J = 17.2 Hz, H-4'); **¹³C-NMR** (75 MHz, CDCl₃): d 195.2, 171.3, 145.1, 136.3, 128.8, 128.7, 128.6, 128.5, 128.4, 128.1, 82.4, 77.4, 71.8, 64.8, 60.3, 39.1; **IR** (film): ? 3429, 3246, 3062, 3033, 2926, 2872, 1790, 1704, 1494, 1453, 1375, 1246, 1219, 1143, 1045, 986, 918, 867, 832, 752, 700.5 cm⁻¹ ; **LMRS (API-ES⁺):** m/z 601 (2M+Na)⁺, 312 (M+Na)⁺, 291(M+2H)⁺, 290 (M+H)⁺.

### rac-(1S,5R,9S)-5-(benzyloxyamino)-9-(tert-butyldimethylsilyloxy)-2-oxabicyclo[4.3.0] non-6-en-3,8-dione (13)

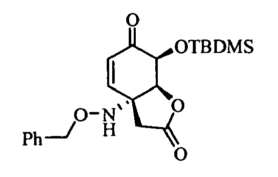

***R_{f}* =** 0.60 (TLC, hexane/AcOEt, 1:1); **yield,** 70% **(*Procedure B*),** 52% **(*Procedure C*);** white solid; **m.p.:** 111-113°C; **¹H-NMR** (200 MHz CDCl₃): d 7.42-7.22 (5H, m, Ph), 6.71 (1H, *part A syst. AB,* J = 10.3 Hz, H-6), 6.13 (1H, *part B syst. AB,* J = 10.3 Hz, H-7), 5.42 (1H, br. s, NH), 4.73 (1H, d, J = 3.7 Hz, H-1), 4.69 (2H, s, OCH₂Ph), 4.35 (1H, d, J = 3.7 Hz, H-9), 2.78 (1H, *part A syst. AB,* J = 17.4 Hz, H-4), 2.48 (1H*, part B syst. AB,* J = 17.4 Hz, H-4'), 0.83 (9H, s, C(CH₃)₃), 0.10 (3H, s, SiCH₃), 0.08 (3H, s, SiCH₃); **¹³C-NMR** (50 MHz, CDCl₃): d 194.7, 173.4, 145.8, 136.4, 128.7, 128.65, 128.6, 128.4, 80.3, 77.6, 73.3, 63.8, 38.3, 25.5, 18.1, -5.2, -5.3; **IR** (film): ? 3239, 2949, 2929, 2855, 1790, 1708, 1471, 1461, 1411, 1367, 1300, 1256, 1159, 1113, 1043, 989, 907, 837, 783, 750 cm⁻¹; **LMRS (API-ES⁺):** m/z 829 (2M+Na)⁺, 807 (2M+H)⁺, 426 (M+Na)⁺, 404 (M+H)⁺.

### Example 4: Preparation of rac-(1S,5R,9S)-5-(benzyloxyamino)-9-(tert-butyldimethylsilyloxy)-8-methylen-2-oxabicyclo[4.3.0]non-6-en-3-one (19)

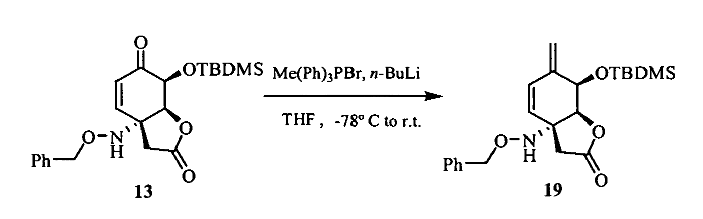

To a stirred suspension of methyltriphenylphosphonium (98 mg, 0.267 mmol) in THF (3 ml) was added dropwise at -78 °C *n*-butyl lithium (0.17 ml, 1.6 M solution in hexane, 0.267 mmol) and the resulting light yellow solution was stirred for 15 min at this temperature. Then, a solution of *rac*-(1*S*,5*R*,9*S*)-5-(benzyloxyamino)-9-(*tert-*butyldimethylsilyloxy)-2-oxabicyclo[4.3.0]non-6-en-3,8-dione (13) (98 mg, 0.243 mmol) in THF (2 ml) was added dropwise and the mixture was stirred for 2.5 h at room temperature. The reaction was concentrated under reduced pressure. The residue was purified by silica gel column chromatography (hexane/AcOEt, 4:1) to give *rac*-(1*S,*5*R,*9*S*)-5-(benzyloxyamino)-9-(*tert*-butyldimethylsilyloxy)-8-methylen-2-oxabicyclo[4.3.0]non-6-en-3-dione (**19**) (33 mg, 36%) as a colourless oil.
***R_{f}*** = 0.55 (TLC, hexane/AcOEt, 2:1); **yield**, 36%; colourless oil; **¹H-NMR** (300 MHz CDCl₃): d 7.35-7.29 (5H, m, Ph), 6.26 (1H, *part A syst. AB,* J = 10 Hz, H-7), 5.70 (1H, *part B syst. AB,* J = 10 Hz, H-6), 5.31 (1H, br. s, NH), 4.23 (1H, s, H-10), 5.19 (1H, s, H-10'), 4.70 (2H, s, OCH₂Ph), 4.54 (2H, m, H-1 and H-9), 2.69 (1H, *part A syst. AB,* J = 17.2 Hz, H-4), 2.50 (1H, *part B syst. AB,* J = 17.2 Hz, H-4'), 0.85 (9H, s, C(CH₃)₃), 0.10 (3H, s, SiCH₃), 0.06 (3H, s, SiCH₃); **¹³C-NMR** (75 MHz, CDCl₃): d 175.0, 141.2, 136.9, 129.0, 128.5, 128.4, 128.1, 127.6, 117.0, 81.5, 77.5, 76.7, 71.6, 64.3, 38.5, 25.6, 18.0, -4.9, -5.0 ; **IR** (film): ? 3379, 3242, 3080, 3063, 3027, 2949, 2929, 2890, 2854, 1784, 1459, 1471, 1453, 1360, 1255, 1108, 1041, 891, 838, 778 cm⁻¹; **LMRS (API-ES⁺):** m/z 601 (2M+Na)⁺, 312 (M+Na)⁺, 291(M+2H)⁺, 290 (M+H)⁺.

### Example 5: Preparation of rac-(1S,5R,9S)-5-(benzyloxyamino)-9-(tert-butyldimethylsilyloxy)-8-hydroxy-8-hydroxymethyl-2-oxabicyclo[4.3.0]non-6-en-3-one (20)

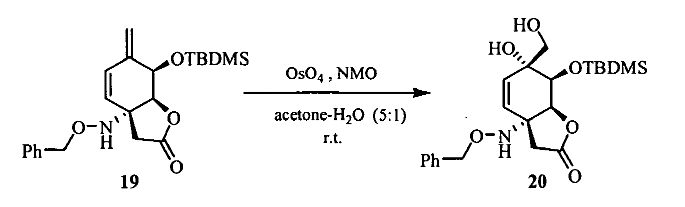

To a solution of *rac*-(1*S*,S*R*,9*S*)-5-(benzyloxyamino)-9-(*tert-*butyldimethylsilyloxy)-8-methylen-2-oxabicyclo[4.3.0]non-6-en-3-one (**19**) (127 mg, 0.316 mmol) in acetone (1.5 ml) was added sequentially, at room temperature, water (0.3 ml), *N*-methylmorpholine *N*-oxide (169 mg, 1.392 mmol) and osmium tetroxide (0.48 ml, 2.5 wt. % solution in 2-methyl-2-propanol, 0.038 mmol). The resulting mixture was stirred at room temperature until the reaction was complete (4 h, TLC monitoring, hexane/AcOEt, 1:1), and then quenched with 10% aqueous Na₂S₂O₃ solution (0.2 ml). After 15 min, the mixture was extracted with AcOEt (5 x 4 ml). The combined organic extracts were dried over Na₂SO₄, filtered and concentrated under reduced pressure. The residue was purified by silica gel column chromatography (hexane/AcOEt, 2:1) to give *rac*-(1*S,*5*R,*9*S*)-5-(benzyloxyamino)-9-(*tert*-butyldimethylsilyloxy)-8-hydroxy-8-hydroxymethyl-2-oxabicyclo[4.3.0]non-6-en-3-one (**20**) (90 mg, 66%) as a colourless oil.
***R_{f}* =** 0.26 (TCL hexane/AcOEt, 1:1); **yield,** 66%; colourless oil; **¹H-NMR** (300 MHz CDCl₃): d 7.40-7.29 (5H, m, Ph), 5.90 (1H, *part A syst. AB,* J = 9.9 Hz, H-6 or H-7), 5.75 (1H, *part B syst. AB,* J = 9.9 Hz, H-7 or H-6), 5.37 (1H, br. s, NH), 4.91 (1H, d, J = 3.4 Hz, H-1 or H-9), 4.73 (1H, *part A syst. AB,* J = 11.3 Hz, OCH₂Ph), 4.69 (1H, *part B syst. AB,* J = 11.3 Hz, OCH₂Ph), 4.20 (1H, d, J = 3.4, H-9 or H-1), 3.80 (1H, *part A syst. AB,* J = 11.2, 4.6 Hz, H-10), 3.50 (1H, *part B syst. AB,* J = 11.2, 8.0 Hz, H-10'), 2.80 (1H, br. s, OH), 2.56 (1H, *part A syst. AB,* J = 17.6 Hz, H-4), 2.49 (1H, *part B syst. AB,* J = 17.6 Hz, H-4'), 2.29 (1H, dd, J = 8.0, 4.6 MHz, OH), 0.86 (9H, s, C(CH₃)₃), 0.14 (3H, s, SiCH₃), 0.12 (3H, s, SiCH₃); **¹³C-NMR** (100 MHz, CDCl₃): d 174.2, 136.2, 130.8, 130.3, 128.8, 128.6, 128.4, 80.4, 77.6, 77.2, 72.3, 72.0, 66.2, 63.8, 38.4, 25.8, 17.9, -4.4, -4.9; **IR** (film): ? 3435, 3027, 2950, 2926, 2884, 2854, 1775, 1494, 1453, 1402, 1361, 1258, 1100, 1052, 839 cm⁻¹ ; **LMRS (API-ES⁺):** m/z 894 (2M+Na)⁺, 458 (M+Na)⁺, 459(M+Na+H)⁺, 436 (M+H)⁺.

### Example 6: Preparation of rac-(1S,5R,9S)-5-(benzyloxyamino)-9-(tert butyldimethylsilyloxy)-8-hydroxy-8-(tert-butyldimethylsilyloxymethyl)-2-oxabicyclo[4.3.0]non-6-en-3-one (21)

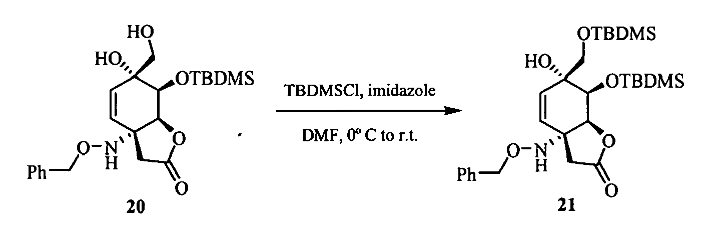

To a solution of *rac*-(1*S*,5*R*,9*S*)-5-(benzyloxyamino)-9-(*tert-*butyldimethylsilyloxy)-8-hydroxy-8-hydroxymethyl-2-oxabicyclo [4.3.0] non-6-en-3 -one (**20**) (66 mg, 0.152 mmol) and imidazole (26 mg, 0.364 mmol) in DMF (0.8 ml) was added at 0 °C a solution of *tert*-butyldimethylsilyl chloride (58 mg, 0.364 mmol) in DMF (0.7 ml). After 26 h at room temperature, the reaction was quenched with H₂O (3 ml) and the mixture extracted with AcOEt (3 x 5 ml). The combined extracts were washed with saturated aqueous CuSO₄ solution (3x10 ml) and brine (2x10 ml), dried over Na₂SO₄, filtered and concentrated under reduced pressure. The residue was purified by silica gel column chromatography (hexane/AcOEt, 6:1) to give *rac*-(1*S*,5*R*,9*S*)-5-(benzyloxyamino)-9-(*tert*-butyldimethylsilyloxy)-8-hydroxy-8-(*tert*-butyldimethylsilyloxymethyl)-2-oxabicyclo[4.3.0]non-6-en-3-one (**21**) (76 mg, 91%) as a colourless oil.
***R_{f}*** = 0.44 (TLC, hexane/AcOEt 3:1); **yield,** 91%; colourless oil; **¹H-NMR** (300 MHz CDCl₃): d 7.35-7.28 (5H, m, Ph), 5.88 (1H, *part A syst. AB,* J = 10.5 Hz, H-6 or H-7), 5.82 (1H, *part B syst. AB,* J = 10.5 Hz, H-7 or H-6), 5.36 (1H, br. s, NH), 4.86 (1H, d, J = 3.4 Hz, H-1 or H-9), 4.74 (1H, *part A syst. AB,* J = 11.7 Hz, OCH₂Ph), 4.69 (1H, *part B syst. AB,* J = 11.7 Hz, OCH₂Ph), 4.10 (1H, d, J = 3.4, H-9 or H-1), 3.70 (1H*, part A syst. AB,* J = 9.8 Hz, H-10), 3.58 (1H, *part B syst. AB,* J = 9.8 Hz, H-10'), 2.88 ( 1H, br.s, OH), 2.58 (1H, *part A syst. AB,* J = 17.3 Hz, H-4), 2.52 (1H, *part B syst. AB,* J = 17.3 Hz, H-4'), 0.92 (9H, s, C(CH₃)₃), 0.84 (9H, s, C(CH₃)₃), 0.13 (3H, s, SiCH₃), 0.10 (3H, s, SiCH₃), 0.09 (3H, s, SiCH₃), 0.07 (3H, s, SiCH₃).

### Synthesis of compounds of formula IV

### Example 7: Preparation of rac-(5R,6S,7S)-1-benzyl-6,7-dihydroxy-6,7-O-(1,1,3,3-tetraisopropyldisiloxane-1,3-diyl)-1-aza-2-oxaspiro[4.5]dec-9-en-3,8-dione (22) and rac-(5R,6S,7S)-6,7-dihydroxy-6,7-O-(1,1,3,3,-tetraisopropyldisiloxane-1,3-diyl)-1-aza-2-oxaspiro[4.5]dec-9-en-3,8-dione (23)

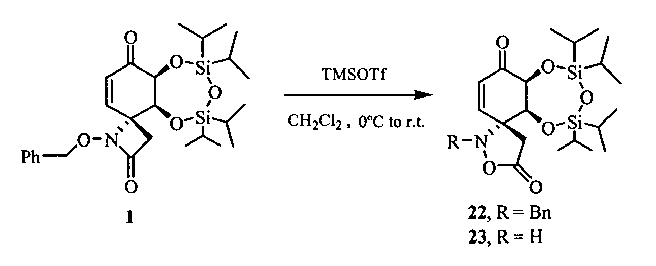

To a stirred solution of *rac*-(4*R*,5*S*,6*S*)-1-benzyloxy-5,6-dihidroxy-5,6-*O*-(1,1,3,3-tetraisopropyldisiloxane-1,3-diyl)-1-azaspiro[3.5]non-8-en-2,7-dione (**1**) (100 mg, 0.188 mmol) in CH₂Cl₂ (3 ml) at 0° C was added trimethylsilyl trifluoromethanesulfonate (41.3 µl, 0.226 mmol). The resulting mixture was stirred at room temperature until the reaction was complete (17 h, TLC monitoring, hexane/AcOEt, 5:1, x4). The reaction was concentrated under reduced pressure. The residue was purified by silica gel column chromatography (hexane/AcOEt, 6:1) to give *rac*-(5*R*,6*S*,7*S*)-1-benzyl-6,7-dihydroxy-6,7-*O*-(1,1,3,3,-tetraisopropyldisiloxane-1,3-diyl)-1-aza-2-oxaspiro[4.5]dec-9-en-3,8-dione (**22**) (15 mg, 14%) and *rac*-(5*R*,6*S*,7*S*)-6,7-dihydroxy-6,7-*O*-(1,1,3,3,-tetraisopropyldisiloxane-1,3-diyl)-1-aza-2-oxaspiro[4.5]dec-9-en-3,8-dione **(23)** (10 mg, 12%), both as an oils.

### rac-(5R,6S,7S)-1-benzyl-6,7-dihydroxy-6,7-O-(1,1,3,3; tetraisopropyldisiloxane-1,3-diyl)-1-aza-2-oxaspiro[4.5]dec-9-en-3,8-dione (22)

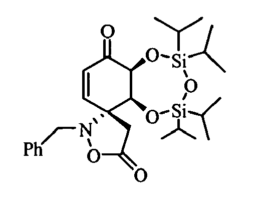

***R_{f}*** = 0.5 (TLC, hexane/AcOEt, 2:1); **yield,** 14%; oil; **¹H-NMR** (300 MHz CDCl₃): d 7.34 (5H, m, Ph), 6.67 (1H, *part A syst. AB,* J = 10.3 Hz, H-9), 6.13 (1H, *part A syst. AB,* J = 10.3 Hz, H-10), 5.09 (1H, br. s, H-6 or H-7), 4.54 (1H, br. s, H-7 or H-6), 4.30 (1H, d, *part A syst. AB,* J = 13.5 Hz, OCH₂Ph), 4.10 (1H*, part B syst. AB,* J = 13.5 Hz, OCH₂Ph), 3.46 (1H, J =17.7 Hz, H-4), 2.91 (1H, J = 17.7 Hz, H-4'), 1.26-0.99 (28H, m, TIPDS); **¹³C-NMR** (100 MHz, CDCl₃): d 194.6, 174.2, 135.5, 131.5, 129.45, 129.0, 128.9, 128.7, 128.6, 128.5, 128.4, 77.5, 75.9, 71.3, 58.5, 37.3, 17.8, 17.7, 17.5, 17.4, 17.2, 17.1, 14.4, 13.7, 13.0; **IR** (film): ? 3430, 2946, 2890, 2866, 1784, 1718, 1627, 1461, 1251, 1185, 1133, 1009, 923, 886, 749, 697 cm⁻¹; **LMRS (API-ES⁺):** m/z 1085 (2M+Na)⁺, 555 (M+Na+H)⁺, 554 (M+Na)⁺, 534 (M+3H)⁺, 533 (M+2H)⁺, 532 (M+H)⁺; **LMRS (EI):** m/z 488 (34), 444 (25), 409 (16), 383 (12), 339 (21), 254 (6), 229 (14), 209 (4), 184 (6), 147 (9), 135 (14),119 (13), 106 (28), 91 (100), 77 (2), 65 (4).

### rac-(5R,6S,7S)-6,7-dihydroxy-6,7-O-(1,1,3,3,-tetraisopropyldisiloxane-1,3-diyl)-1-aza-2-oxaspiro[4.5]dec-9-en-3,8-dione(23)

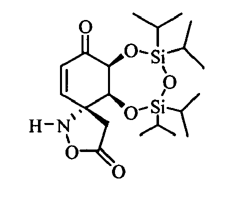

***R_{f} =*** 0.25 (TLC, hexane/AcOEt, 2:1); **yield,** 12%; oil; **¹H-NMR** (300 MHz CDCl₃): d 6.88 (2H, br. s, NH and H-8 or H-9), 6.14 (1H, d, J = 10.2 Hz, H-9 or H-8), 4.83- 4.72 (1H, br. s, H-6 or H-7), 4.38 (1H, br. s, H-7 or H-6), 3.44 (1H, d, *part A syst. AB, J* = 18.4 Hz, H-4), 2.67 (1H*, part B syst. AB,* J = 18.4 Hz, H-4'), 1.26-1.00 (28H, m, TIPDS); **¹³C-NMR** (100 MHz, CDCl₃): d 193.8, 176.7, 137.9, 128.5, 75.4, 67.1, 37.2, 17.4, 17.3, 17.2, 17.1, 17.0, 13.9, 13.2, 12.7, 2.0; **IR** (film): ? 3435, 3228, 2947, 2890, 2869, 1793, 1707, 1465, 1251, 1185, 1141, 1095, 1020, 923, 885, 699 cm⁻¹; **LMRS (API-ES⁺):** m/z 465 (M+Na+H)⁺, 464 (M+Na)⁺, 442 (M+H)⁺. **LMRS (EI):** m/z 441 (M⁺, 2), 409 (19), 398 (55), 370 (10), 354 (100), 339 (8), 327 (8), 308 (6), 295 (3), 259 (6), 235 (18), 207 (21),193 (17), 175 (19),164 (35),147 (28),135 (56),119 (48),105 (30), 91 (14), 77 (7), 65 (9).

### Example 8: Reaction of rac-(4R,5S,6S,7R)-1-benzyloxy-5,6,7-trihydroxy-7-(hydroxymethyl)-5,6-O-(1,1,3,3-tetraisopropyldisiloxane-1,3-diyl)-1-azaspiro[3.5]non-8-en-2-one (24), 4-methoxybenzyl chloride and sodium hydride

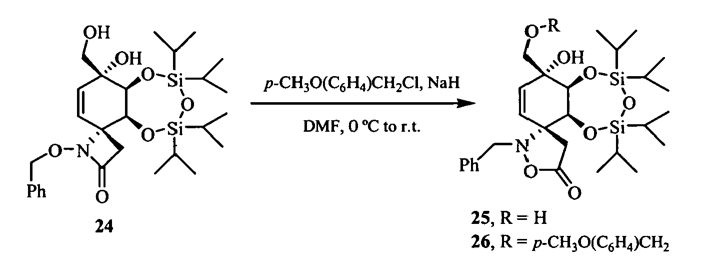

To a solution of *rac-*(4*R,*5*S,*6*S,*7*R)-*1-benzyloxy-5,6,7-trihydroxy-7-(hydroxymethyl)-5,6-*O*-(1,1,3,3-tetraisopropyldisiloxane-1,3-diyl)-1-azaspiro[3.5]non-8-en-2-one (**24**) (76 mg, 0.135 mmol) in dry DMF (0.4 ml) was added at 0 °C NaH (7 mg, 0.162 mmol), the mixture was stirred for 30 min and then was added 4-methoxybenzyl chloride (24 µl, 0.175 mmol) and the resulting mixture was stirred at room temperature. After 2.5 h, the reaction was quenched with cold water (1 ml) and saturated aqueous NH₄Cl solution (2 ml). The mixture was extracted with CH₂Cl₂ (3 x 2 ml). The combined organic extracts were washed with H₂O (2 ml), dried over Na₂SO₄, filtered and concentrated under reduced pressure. The residue was purified by silica gel column chromatography (hexano/AcOEt, 6:1) to give *rac*-(5*R,6S,7S,8R*)-1-benzyl-6,7,8-trihydroxy-8-(hydroxymethyl)-6,7-*O*-(1,1,3,3-tetraisopropyldisiloxane-1,3-diyl)-1-aza-2-oxaspiro[4.5]dec-9-en-3-one (**25**) (14 mg, 18%), as a white solid, *rac*-(5*R,*6*S,*7*S,*8*R*)-1-benzyl-6,7,8-trihydroxy-8-[(4-methoxybenzyloxy)methyl]-6,7-*O*-(1,1,3,3-tetraisopropyldisiloxane-1,3-diyl)-1-aza-2-oxaspiro[4.5]dec-9-en-3-one (**26**) (6 mg, 6%), as a yellow oil, and diol **24** (19 mg, 25%).

### rac-(5R,6S,7S,8R)-1-benzyl-6,7,8-trihydroxy-8-(hydroxymethyl)-6,7-O-(1,1,3,3-tetraisopropyldisiloxane-1,3-diyl)-1-aza-2-oxaspiro[4.5]dec-9-en-3-one (25)

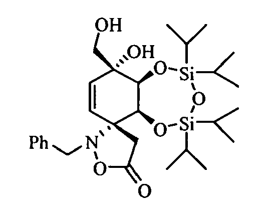

***R_{f}*** = 0.19 (TLC, hexano/AcOEt, 3:1), 0.45 (TLC, hexano/AcOEt, 1:1); **yield,** 18%; white solid; **¹H-NMR** (200 MHz, CDCl₃): δ 7.33 (5H, br. s, Ph), 6.05 (1H, *part A syst. AB,* J = 9.9 Hz, H-10), 5.45 (1H, *part B syst. AB,* J = 9.9 Hz, H-9), 4.88 (1H, d, J = 3.5 Hz, H-6), 4.71 (2H, s, NCH₂Ph), 4.29 (1H, d, J = 3.5 Hz, H-7), 4.03 (1H, *part A syst. AB, J* = 11.3 Hz, H-11), 3.58 (1H, *part B syst. AB,* J = 11.3 Hz, H-11'), 2.63 (1H, br. s, OH), 2.62 (1H, *part A syst. AB,* J = 17.4 Hz, H-4), 2.43 (1H, *part B syst. AB,* J = 17.4 Hz, H-4'), 1.58 (1H, br. s, OH), 1.16-0.88 (28H, m, TIPDS); **¹³C-NMR** (75 MHz, CDCl₃): δ 175.5, 136.9, 132.1, 128.7, 128.4, 128.1, 126.5, 79.6, 77.7, 72.6, 70.0, 65.4, 63.6, 38.4, 17.4, 17.3, 17.25, 17.2, 17.1, 17.0, 13.3, 13.1, 12.8, 12.75; **IR** (film): ν 3405, 2945, 2862, 1787, 1655, 1511, 1461, 1383, 1355, 1244, 1147, 1111, 1060, 1033, 919, 883 cm⁻¹; **LRMS (API-ES⁺):** m/z 1149 (2M+Na)⁺, 1127 (2M+H)⁺, 586 (M+Na)⁺, 564 (M+H)⁺, 546 (M-17)⁺.

### rac-(5R,6S,7S,8R)-1-benzyl-6,7,8-trihydroxy-8-[(4-methoxybenzyloxy)methyl]-6,7-O-(1,1,3,3-tetraisopropyldisiloxane-1,3-diyl)-1-aza-2-oxaspiro[4.5]dec-9-en-3-one (26)

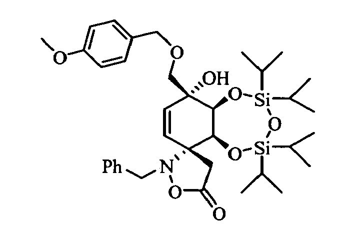

***R_{f}*** = 0.36 (TLC, hexano/AcOEt, 3:1), 0.73 (TLC, hexano/AcOEt, 1:1); **yield,** 6%; yellow oil; **¹H-NMR** (200 MHz, CDCl₃): δ 7.41-7.28 (5H, m, Ph), 7.21 (2H, d, J = 8.6 Hz, *p-*CH₃OC₆H₄), 6.84 (2H, d, J = 8.6 Hz,*p*-CH₃OC₆H₄), 5.85 (1H, *part A syst. AB*, J = 10.0 Hz, H-9 or H-10), 5.60 (1H,*part B syst. AB,* J = 10.0 Hz, H-10 or H-9), 4.94 (1H, d, J = 3.4 Hz, H-6 or H-7), 4.69 (2H, s, NCH₂Ph), 4.68 (1H, *part A syst. AB,* J = 8.6 Hz, OCH₂Ar), 4.58 (1H, *part B syst. AB,* J = 8.6 Hz, OCH₂Ar), 4.01 (1H, s, H-7 or H-6), 3.99 (1H, *part A syst. AB,* J = 11.3 Hz, H-11), 3.86 (1H, *part B syst. AB,* J = 11.3 Hz, H-11'), 3.78 (3H, s, OCH₃), 2.66 (1H, *part A syst. AB,* J = 17.7 Hz, H-4), 2.41 (1H, *part B syst. AB, J* = 17.7 Hz, H-4'), 1.58 (1H, br. s, OH), 1.20-0.76 (28H, m, TIPDS); **¹³C-NMR** (75 MHz, CDCl₃): δ 176.0, 159.4, 137.1, 132.7, 130.4, 129.1, 128.4, 128.3, 128.0, 126.3, 80.4, 76.3, 75.4, 70.3, 66.8, 64.3, 63.7, 55.2, 38.6, 17.5, 17.45, 17.4, 17.35, 17.3, 17.25, 13.3, 13.2, 12.9, 12.85; **IR** (film): v 3420, 3028, 2945, 2889, 2867, 1785, 1609, 1514, 1464, 1389, 1302, 1249, 1147, 1090, 1066, 1033, 1010, 922, 885, 800, 696 cm⁻¹ ; **LRMS (API-ES⁺):** m/z 1389 (2M+Na)⁺, 706 (M+Na)⁺, 684 (M+H)⁺.

## Claims

1. A process for rearranging the spirolactam of a compound of formula I 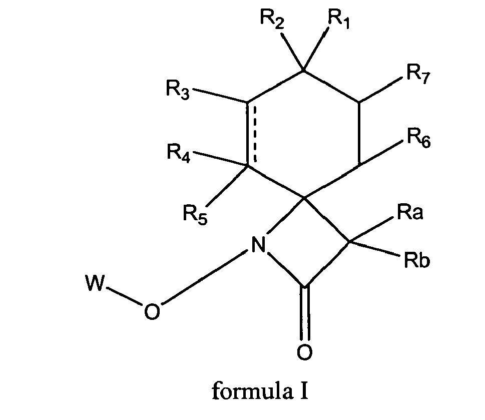 wherein
R₁ is selected from the group consisting of-H, -OH and -OPr₁;
R₂ is selected from the group consisting of -H, -CH₂OH and -CH₂OPr₂; or
R₁ and R₂ together are =O, substituted or unsubstituted alkenyl, -CH₂-O-, or-O-Pr_{cy}-O-CH₂-;
R₃ is selected from the group consisting of-H, -OH, -OPr₃ and =O;
R₄ is selected from the group consisting of -H, cyano, substituted or unsubstituted alkyl, -OPrl₄, -OPr₄, -OH, substituted or unsubstituted alkenyl and substituted or unsubstituted alkynyl;
the dotted lines represent an optional additional bond;
if the double bond is present R₅ is not present;
if the bond between C8 and C9 is a single bond, R₅ is selected from the group consisting of-H, -OH and -OPr₅; or
R₄ and R₅ together are =O; or
R₃ and R₅ or R₃ and R₂ together are -O-, forming an epoxide ring;
R₆ is selected from -H, -OH, -OPrl₆, -OPr₆ and =O;
R₇ is selected from the group consisting of -H, -OH, -OPr₇ and =O; or
R₇ and R₆ together are -O-Pr_{cy}-O-;
Ra and Rb are each independently selected from the group consisting of -H, -OH, -OPrab, substituted or unsubstituted alkyl, substituted or unsubstituted amino and halogen;
Pr₁, Pr₂, Pr₃, Pr₄, Pr₅, Pr₆, Pr₇, Prey and Prab, are, the same or different, hydroxyl protecting groups;
Prl₆ and Prl₄ are acid labile hydroxyl protecting groups;
W is selected from the group consisting of -H, substituted or unsubstituted alkyl, substituted or unsubstituted aryl, substituted or unsubstituted aralkyl, substituted or unsubstituted heterocyclyl and substituted or unsubstituted alkenyl;
or its tautomers, salts or solvates thereof;
wherein
A) if R₄ is -OH or -OPrl₄ and the compound of formula I is reacted with an acid reagent, a compound of formula II is formed 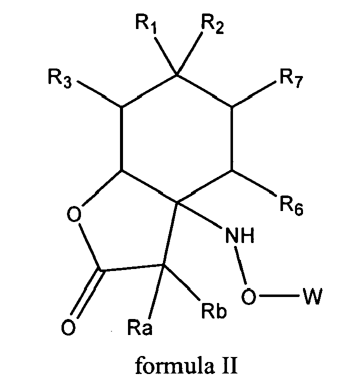 wherein R₁, R₂, R₃, R₆, R₇, Ra, Rb and W are as defined in formula I;
or its tautomers, salts or solvates thereof; or
B) if R₆ is -OH or -OPrl₆ and the compound of formula I is reacted with an acid reagent, a compound of formula III is formed 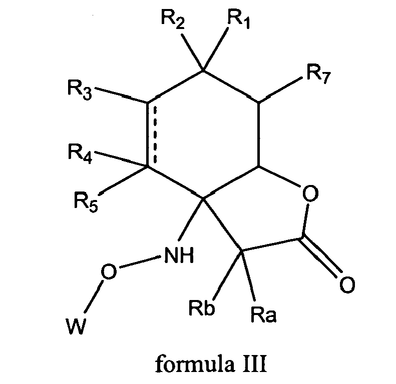 wherein R₁, R₂, R₃, R₄, R₅, R₇, Ra, Rb and W are as defined in formula I;
or its tautomers, salts or solvates thereof; or
C) if R₄ is not -OH or -OPrl₄, and R₆ is not -OH or -OPrl₆,and the compound of formula I is reacted with an acid reagent or with halogen hydride, a compound of formula IV is formed 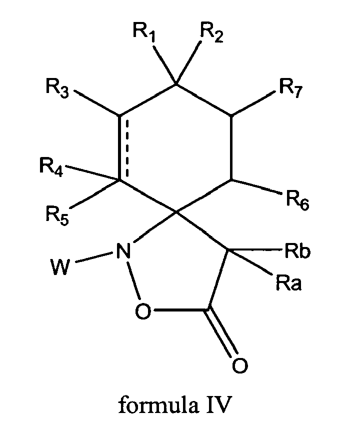 wherein R₁, R₂, R₃, R₄, R₅, R₆, R₇, Ra, Rb and W are as defined in formula I; or its tautomers, salts or solvates thereof.

2. Process according to claim 1 which comprises reacting a compound of formula I as defined in claim 1, wherein R₄ is selected from the group consisting of -OH and -OPrl₄, and R₁, R₂, R₃, R₅, R₆, R₇, Ra, Rb and W are as defined in claim 1 with an acid reagent to yield a compound of formula II as defined in claim 1.

3. Process according to claim 1 which comprises reacting a compound of formula I as defined in claim 1, wherein R₆ is selected from the group consisting of -OH and -OPrl₆, and R₁, R₂, R₃, R₄, R₅, R₇, Ra, Rb and W are as defined in claim 1 with an acid reagent to yield a compound of formula III as defined in claim 1.

4. Process according to any of the previous claims, wherein Prl₆ and Prl₄ are independently selected from the group consisting of silyl ethers of formula -Si(R')₃ or esters of formula -C(=O)R'; wherein R' represents a substituent selected from the group consisting of substituted of unsubstituted alkyl, substituted of unsubstituted alkenyl, substituted of unsubstituted alkynyl, substituted of unsubstituted aryl and substituted of unsubstituted aralkyl.

5. Process according to claim 4, wherein Prl₆ and Prl₄ are independently selected from the group consisting of trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether, acetate ester, benzoate ester, pivalate ester, chloroacetate ester and levulinate ester.

6. A Process according to any of the previous claims, wherein said acid reagent is selected form the group consisting of silyl triflates, preferably TMSOTf, and diluted acids, preferably diluted sulphuric acid, diluted hydrochloric acid or diluted acetic acid.

7. Process according to claim 1 which comprises reacting a compound of formula I as defined in claim 1, wherein R₄ is not -OH or -OPrl₄, R₆ is not -OH or -OPrl₆, and R₁, R₂, R₃, R₅, R₇, Ra, Rb and W are as defined in claim 1 with an acid reagent or with a halogen hydride, preferably sodium hydride, to yield a compound of formula IV as defined in claim 1.

8. Process according to claim 7 which comprises reacting the compound of formula I, wherein one or more of R₁, R₂, R₃, R₅, R₇, Ra or Rb are OH, its tautomers, salts or solvates thereof, with a halogen hydride and an electrophile.

9. Process according to claim 8, wherein said electrophile has the general formula R₈X, wherein X is a halogen atom, and R₈ is selected from the group consisting of substituted or unsubstituted alkyl, substituted or unsubstituted alkenyl, substituted or unsubstituted aryl, substituted or unsubstituted aralkyl, substituted or unsubstituted heterocyclyl, -C(O)R₉, -C(O)OR₉ and -C(O)NR₉R₁₀, preferably substituted or unsubstituted aralkyl, wherein each of R₉ and R₁₀ is independently selected from the group consisting of substituted or unsubstituted alkyl, substituted or unsubstituted alkenyl, substituted or unsubstituted aryl, substituted or unsubstituted aralkyl and substituted or unsubstituted heterocyclyl.

10. A compound of formula II as defined in claim 1 or its tautomers, salts or solvates thereof.

11. Compound according to claim 10, wherein R₃ is -OH or -OPr₃.

12. Compound according to any of claims 10 and 11, wherein R₇ is -OH or -OPr₇.

13. Compound according to any of claims 9 and 10, wherein at least one of Pr₃ and Pr₇ is also an acid labile hydroxyl protecting group.

14. Compound according to claim 10, wherein R₆ and R₇ together are -O-Prcy-O-

15. Compound according to any of claims 10-14, wherein W in -CH₂-Ph.

16. Compound according to claim 10 of formula IIb 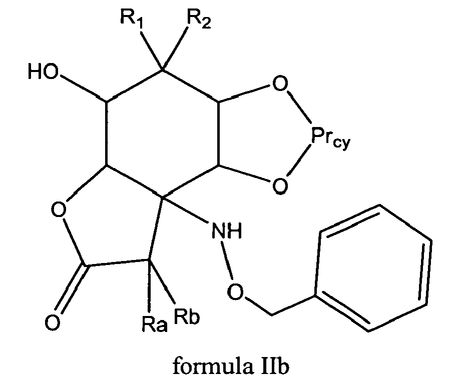 wherein R₁, R₂, Ra, Rb and Prcy are as defined in claim 1;
or its tautomers, salts or solvates thereof.

17. Compound according to claim 10, wherein the compound of formula II is selected from the group consisting of *rac*-(1*R*,5*R*,6*S*,7*S*,8*S*,9*R*)-5-(benzyloxyamino)-8-(benzoyloxymethyl)-9-(*tert*-butyldimethylsilyloxy)-6,7,8-trihydroxy-6,7-*O*-(1,1,3,3-tetraisopropyldisiloxano-1,3-diyl)-2-oxabicyclo[4.3.0]nonan-3-one and *rac-*(*1R,5R,6S,7S,8S,9R*)-5-(benzyloxyamino)-8-(benzoyloxymethyl) -6,7,8,9-tetrahydroxy -6,7-O-(1,1,3,3-tetraisopropyldisiloxane-1,3-diyl)-2-oxabicyclo[4.3.0]nonan-3-one, or its enantiomers, tautomers, salts or solvates thereof.

18. A compound of formula III as defined in claim 1 or its tautomers, salts or solvates thereof.

19. Compound according to claim 18, wherein R₃ is -OH or -OPr₃.

20. Compound according to any of claims 18 and 19, wherein R₇ is -OH or -OPr₇.

21. Compound according to any of claims 19 and 20, wherein at least one of Pr₃ and Pr₇ is also an acid labile hydroxyl protecting group.

22. Compound according to any of claims 18-21, wherein W in -CH₂-Ph.

23. Compound according to claim 18 of IIIb 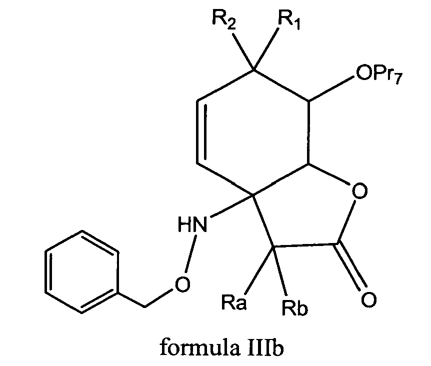 wherein R₁, R₂, Ra, Rb and Pr₇ are as defined in claim 1;
or its tautomers, salts or solvates thereof

24. Compound according to 18, wherein the compound of formula III is selected from the group consisting of rac-(1S,5R,9S)-5-(benzyloxyamino)-9-hydroxy-2-oxabicyclo[4.3.0]non-6-en-3,8-dione, rac-(1S,5R,9S)-5-(benzyloxyamino)-9-(tert-butyldimethylsilyloxy)-2-oxabicyclo[4.3.0]non-6-en-3,8-dione, rac-(1S,5R,9S)-5-(benzyloxyamino)-9-(tert-butyldimethylsilyloxy)-8-methylen-2-oxabicyclo[4.3.0]non-6-en-3-one, rac-(1S,5R,9S)-5-(benzyloxyamino)-9-(tert-butyldimethylsilyloxy)-8-hydroxy-8-hydroxymethyl-2-oxabicyclo[4.3.0]non-6-en-3-one and rac-(1S,5R,9S)-5-(benzyloxyamino)-9-(tert-butyldimethylsilyloxy)-8-hydroxy-8-(tert-butyldimethylsilyloxy methyl)-2-oxabicyclo[4.3.0]non-6-en-3-one, or its enantiomers, tautomers, salts or solvates thereof.

25. A compound of formula IV as defined in claim 1 or its tautomers, salts or solvates thereof.

26. Compound according to claim 25 of formula IVb 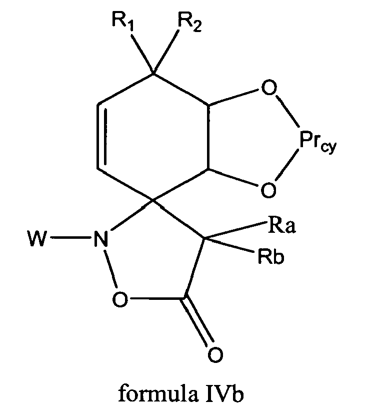 wherein R₁, R₂, Ra, Rb, Prcy and W are as defined in claim 1;
or its tautomers, salts or solvates thereof

27. Compound according to claim 25, wherein the compound of formula IV is selected from the group consisting of rac-(5R,6S,7S,8R)-1-benzyl-6,7,8-trihydroxy-8-[(4-methoxybenzyloxy)methyl] -6,7-O-(1,1,3,3-tetraisopropyldisiloxane-1,3-diyl)-1-aza-2-oxaspiro[4.5]dec-9-en-3-one, rac-(5R,6S,7S,8R)-l-benzyl-6,7,8-trihydroxy-8-(hydroxymethyl)-6,7-O-(1,1,3,3-tetraisopropyldisiloxane-1,3-diyl)-1-aza-2-oxaspiro[4.5]dec-9-en-3-one, rac-(SR,6S,7S)-1-benzyl-6,7-dihydroxy-6,7-O-(1,1,3,3,-tetraisopropyl disiloxane-1,3-diyl)-1-aza-2-oxaspiro[4.5]dec-9-en-3,8-dione and rac-(5R,6S,7S)-6,7-dihydroxy-6,7-O-(1,1,3,3,-tetraisopropyldisiloxane-1,3-diyl)-1-aza-2-oxaspiro[4.5]dec-9-en-3,8-dione, or its enantiomers, tautomers, salts or solvates thereof.
